# EUROPEAN PATENT SPECIFICATION

(11) **EP 1 147 174 B1**
(45) Date of publication and mention of the grant of the patent: **14.02.2007**
(21) Application number: 99965105.2
(22) Date of filing: 06.12.1999
(51) Int. Cl.: C12N 1/10, C12N 1/12, C12N 9/00, C12N 15/06, C12N 15/09, C12N 15/12, C12N 15/30

(54) **DESATURASES AND METHODS OF USING THEM FOR SYNTHESIS OF POLYUNSATURATED FATTY ACIDS**
DESATURASEN UND VERFAHREN IHRER ANWENDUNG ZUR SYNTHESE VON MEHRFACH UNGESÄTTIGTEN FETTSÄUREN
DESATURASES ET LEURS METHODES D'UTILISATION POUR LA SYNTHESE D'ACIDES GRAS POLYINSATURES

(30) Priority: 07.12.1998 US 111301 P
(43) Date of publication of application: 24.10.2001
(73) Proprietor: WASHINGTON STATE UNIVERSITY RESEARCH FOUNDATION, Pullman, WA 99163 (US)
(72) Inventor: BROWSE, John, A., Palouse, WA 99161 (US); WALLIS, James, G., Moscow, ID 83843 (US); WATTS, Jennifer, L., Moscow, ID 83843 (US)
(74) Representative: Gowshall, Jonathan Vallance
(86) International application number: PCT/US1999/028655
(87) International publication number: WO 2000/034439

(56) References cited:
- WO-A-96/21022
- US-A- 5 057 419
- SPYCHALLA ET AL: "Identification of an animal omega-3 fatty acid desaturase by heterologous gene expression in Arabidopsis" PROCEEDINGS OF THE NATIONAL ACADEMY OF SCIENCES OF USA, NATIONAL ACADEMY OF SCIENCE. WASHINGTON, US, vol. 94, February 1997 (1997-02), pages 1142-1147, XP002099449 ISSN: 0027-8424
- SAYANOVA ET AL: "Expression of a borage desaturase cDNA containing an N-terminal cytochrome b5 domain results in the accumulation of high levels of delta-6-desaturated fatty acids in transgenic tobacco" PROCEEDINGS OF THE NATIONAL ACADEMY OF SCIENCES OF USA, NATIONAL ACADEMY OF SCIENCE. WASHINGTON, US, vol. 94, April 1997 (1997-04), pages 4211-4216, XP002099447 ISSN: 0027-8424
- WALLIS JAMES G ET AL: "The DELTA8-desaturase of Euglena gracilis: An alternate pathway for synthesis of 20-carbon polyunsaturated fatty acids" ARCHIVES OF BIOCHEMISTRY AND BIOPHYSICS, vol. 365, no. 2, 15 May 1999 (1999-05-15), pages 307-316, XP002291433 ISSN: 0003-9861

### SEQUENCE LISTING

<110> Browse, John et al.
<120> Desaturases and Methods of Using Them for Synthesis of Polyunsaturated Fatty Acids
<130> 53860
<140>
   <141>
<150> 60/111,301
   <151> 1998-12-07
<160> 13
<170> PatentIn Ver. 2.0
<210> 1
   <211> 1461
   <212> DNA
   <213> Caenorhabditis elegans
<400> 1
   gaattttcaa tcctccttgg gtcccaccgc tgtgatatca aaatggtatt acgagagcaa 60
   gagcatgagc cattcttcat taaaattgat ggaaaatggt gtcaaattga cgatgctgtc 120
   ctgagatcac atccaggtgg tagtgcaatt actacctata aaaatatgga tgccactacc 180
   gtattccaca cattccatac tggttctaaa gaagcgtatc aatggctgac agaattgaaa 240
   aaagagtgcc ctacacaaga accagagatc ccagatatta aggatgaccc aatcaaagga 300
   attgatgatg tgaacatggg aactttcaat atttctgaga aacgatctgc ccaaataaat 360
   aaaagtttca ctgatctacg tatgcgagtt cgtgcagaag gacttatgga tggatctcct 420
   ttgttctaca ttagaaaaat tcttgaaaca atcttcacaa ttctttttgc attctacctt 480
   caataccaca catattatct tccatcagct attctaatgg gagttgcgtg gcaacaattg 540
   ggatggttaa tccatgaatt cgcacatcat cagttgttca aaaacagata ctacaatgat 600
   ttggccagct atttcgttgg aaacttttta caaggattct catctggtgg ttggaaagag 660
   cagcacaatg tgcatcacgc agccacaaat gttgttggac gagacggaga tcttgattta 720
   gtcccattct atgctacagt ggcagaacat ctcaacaatt attctcagga ttcatgggtt 780
   atgactctat tcagatggca acatgttcat tggacattca tgttaccatt cctccgtctc 840
   tcgtggcttc ttcagtcaat catttttgtt agtcagatgc caactcatta ttatgactat 900
   tacagaaata ctgcgattta tgaacaggtt ggtctctctt tgcactgggc ttggtcattg 960
   ggtcaattgt atttcctacc cgattggtca actagaataa tgttcttcct tgtttctcat 1020
   cttgttggag gtttcctgct ctctcatgta gttactttca atcattattc agtggagaag 1080
   tttgcattga gctcgaacat catgtcaaat tacgcttgtc ttcaaatcat gaccacaaga 1140
   aatatgagac ctggaagatt cattgactgg ctttggggag gtcttaacta tcagattgag 1200
   caccatcttt tcccaacgat gccacgacac aacttgaaca ctgttatgcc acttgttaag 1260
   gagtttgcag cagcaaatgg tttaccatac atggtcgacg attatttcac aggattctgg 1320
   cttgaaattg agcaattccg aaatattgca aatgttgctg ctaaattgac taaaaagatt 1380
   gcctagatta cgattaatta atcaatttat tttcatgttc tattcgtgtg ttttaatatt 1440
   ttccaaattt ttacctattc c 1461
<210> 2
   <211> 447
   <212> PRT
   <213> Caenorhabditis elegans
<400> 2
   Met Val Leu Arg Glu Gln Glu His Glu Pro Phe Phe Ile Lys Ile Asp
   1 5 10 15
   Gly Lys Trp Cys Gln Ile Asp Asp Ala Val Leu Arg Ser His Pro Gly
               20                  25                  30
   Gly Ser Ala Ile Thr Thr Tyr Lys Asn Met Asp Ala Thr Thr Val Phe
           35                  40                  45
   His Thr Phe His Thr Gly Ser Lys Glu Ala Tyr Gln Trp Leu Thr Glu
       50                  55                  60
   Leu Lys Lys Glu Cys Pro Thr Gln Glu Pro Glu Ile Pro Asp Ile Lys
   65                  70                  75                  80
   Asp Asp Pro Ile Lys Gly Ile Asp Asp Val Asn Met Gly Thr Phe Asn
                   85                  90                  95
   Ile Ser Glu Lys Arg Ser Ala Gln Ile Asn Lys Ser Phe Thr Asp Leu
               100                 105                 110
   Arg Met Arg Val Arg Ala Glu Gly Leu Met Asp Gly Ser Pro Leu Phe
           115                 120                 125
   Tyr Ile Arg Lys Ile Leu Glu Thr Ile Phe Thr Ile Leu Phe Ala Phe
       130                 135                 140
   Tyr Leu Gln Tyr His Thr Tyr Tyr Leu Pro Ser Ala Ile Leu Met Gly
   145                 150                 155                 160
   Val Ala Trp Gln Gln Leu Gly Trp Leu Ile His Glu Phe Ala His His
                   165                 170                 175
   Gln Leu Phe Lys Asn Arg Tyr Tyr Asn Asp Leu Ala Ser Tyr Phe Val
               180                 185                 190
   Gly Asn Phe Leu Gln Gly Phe Ser Ser Gly Gly Trp Lys Glu Gln His
           195                 200                 205
   Asn Val His His Ala Ala Thr Asn Val Val Gly Arg Asp Gly Asp Leu
       210                 215                 220
   Asp Leu Val Pro Phe Tyr Ala Thr Val Ala Glu His Leu Asn Asn Tyr
   225                 230                 235                 240
   Ser Gln Asp Ser Trp Val Met Thr Leu Phe Arg Trp Gln His Val His
                   245                 250                 255
   Trp Thr Phe Met Leu Pro Phe Leu Arg Leu Ser Trp Leu Leu Gln Ser
               260                 265                 270
   Ile Ile Phe Val Ser Gln Met Pro Thr His Tyr Tyr Asp Tyr Tyr Arg
           275                 280                 285
   Asn Thr Ala Ile Tyr Glu Gln Val Gly Leu Ser Leu His Trp Ala Trp
       290                 295                 300
   Ser Leu Gly Gln Leu Tyr Phe Leu Pro Asp Trp Ser Thr Arg Ile Met
   305                 310                 315                 320
   Phe Phe Leu Val Ser His Leu Val Gly Gly Phe Leu Leu Ser His Val
                   325                 330                 335
   Val Thr Phe Asn His Tyr Ser Val Glu Lys Phe Ala Leu Ser Ser Asn
               340                 345                 350
   Ile Met Ser Asn Tyr Ala Cys Leu Gln Ile Met Thr Thr Arg Asn Met
           355                 360                 365
   Arg Pro Gly Arg Phe Ile Asp Trp Leu Trp Gly Gly Leu Asn Tyr Gln
       370                 375                 380
   Ile Glu His His Leu Phe Pro Thr Met Pro Arg His Asn Leu Asn Thr
   385                 390                 395                 400
   Val Met Pro Leu Val Lys Glu Phe Ala Ala Ala Asn Gly Leu Pro Tyr
                   405                 410                 415
   Met Val Asp Asp Tyr Phe Thr Gly Phe Trp Leu Glu Ile Glu Gln Phe
               420                 425                 430
   Arg Asn Ile Ala Asn Val Ala Ala Lys Leu Thr Lys Lys Ile Ala
           435                 440                 445
<210> 3
   <211> 1275
   <212> DNA
   <213> Euglena gracilis
<400> 3
   attttttttc gaaatgaagt caaagcgcca agcgctatcc cccttacaat tgatggaaca 60
   aacatatgat gtggtcaatt tccaccctgg tggtgcggaa attatagaga attaccaagg 120
   aagggatgcc actgatgcct tcatggttat gcactttcaa gaagccttcg acaagctcaa 180
   gcgcatgccc aaaatcaatc ccagttttga gttgccaccc caggctgcag tgaatgaagc 240
   tcaagaggat ttccggaagc tccgagaaga gttgatcgca actggcatgt ttgatgcctc 300
   ccccctctgg tactcataca aaatcagcac cacactgggc cttggagtgc tgggttattt 360
   cctgatggtt cagtatcaga tgtatttcat tggggcagtg ttgcttggga tgcactatca 420
   acagatgggc tggctttctc atgacatttg ccaccaccag actttcaaga accggaactg 480
   gaacaacctc gtgggactgg tatttggcaa tggtctgcaa ggtttttccg tgacatgttg 540
   gaaggacaga cacaatgcac atcattcggc aaccaatgtt caagggcacg accctgatat 600
   tgacaacctc ccccccttag cctggtctga ggatgacgtc acacgggcgt caccgatttc 660
   ccgcaagctc attcagttcc agcagtacta tttcttggtc atctgtatct tgttgcggtt 720
   catttggtgt ttccagtgcg tgttgaccgt gcgcagtttg aaggacagag ataaccaatt 780
   ctatcgctct cagtataaga aggaggccat tggcctcgcc ctgcactgga ccttgaaggc 840
   cctgttccac ttattcttta tgcccagcat cctcacatcg ctgttggtgt ttttcgtttc 900
   ggagctggtt ggcggcttcg gcattgcgat cgtggtgttc atgaaccact acccactgga 960
   gaagatcggg gacccagtct gggatggcca tggattctcg gttggccaga tccatgagac 1020
   catgaacatt cggcgaggga ttatcacaga ttggtttttc ggaggcttga attaccagat 1080
   tgagcaccat ttgtggccga ccctccctcg ccacaacctg acagcggtta gctaccaggt 1140
   ggaacagctg tgccagaagc acaacctgcc gtatcggaac ccgctgcccc atgaagggtt 1200
   ggtcatcctg ctgcgctatc tggcggtgtt cgcccggatg gcggagaagc aacccgcggg 1260
   gaaggctcta taagg                                                  1275
<210> 4
   <211> 422
   <212> PRT
   <213> Euglena gracilis
<400> 4
   Met Lys Ser Lys Arg Gln Ala Leu Ser Pro Leu Gln Leu Met Glu Gln
    1               5                  10                  15
   Thr Tyr Asp Val Ser Ala Trp Val Asn Phe His Pro Gly Gly Ala Glu
               20                  25                  30
   Ile Ile Glu Asn Tyr Gln Gly Arg Asp Ala Thr Asp Ala Phe Met Val
           35                  40                  45
   Met His Phe Gln Glu Ala Phe Asp Lys Leu Lys Arg Met Pro Lys Ile
       50                  55                  60
   Asn Pro Ser Phe Glu Leu Pro Pro Gln Ala Ala Val Asn Glu Ala Gln
   65                  70                  75                  80
   Glu Asp Phe Arg Lys Leu Arg Glu Glu Leu Ile Ala Thr Gly Met Phe
                   85                  90                  95
   Asp Ala Ser Pro Leu Trp Tyr Ser Tyr Lys Ile Ser Thr Thr Leu Gly
               100                 105                 110
   Leu Gly Val Leu Gly Tyr Phe Leu Met Val Gln Tyr Gln Met Tyr Phe
           115                 120                 125
   Ile Gly Ala Val Leu Leu Gly Met His Tyr Gln Gln Met Gly Trp Leu
       130                 135                 140
   Ser His Asp Ile Cys His His Gln Thr Phe Lys Asn Arg Asn Trp Asn
   145                 150                 155                 160
   Asn Leu Val Gly Leu Val Phe Gly Asn Gly Leu Gln Gly Phe Ser Val
                   165                 170                 175
   Thr Cys Trp Lys Asp Arg His Asn Ala His His Ser Ala Thr Asn Val
               180                 185                 190
   Gln Gly His Asp Pro Asp Ile Asp Asn Leu Pro Pro Leu Ala Trp Ser
           195                 200                 205
   Glu Asp Asp Val Thr Arg Ala Ser Pro Ile Ser Arg Lys Leu Ile Gln
       210                 215                 220
   Phe Gln Gln Tyr Tyr Phe Leu Val Ile Cys Ile Leu Leu Arg Phe Ile
   225                 230                 235                 240
   Trp Cys Phe Gln Cys Val Leu Thr Val Arg Ser Leu Lys Asp Arg Asp
                   245                 250                 255
   Asn Gln Phe Tyr Arg Ser Gln Tyr Lys Lys Glu Ala Ile Gly Leu Ala
               260                 265                 270
   Leu His Trp Thr Leu Lys Ala Leu Phe His Leu Phe Phe Met Pro Ser
           275                 280                 285
   Ile Leu Thr Ser Leu Leu Val Phe Phe Val Ser Glu Leu Val Gly Gly
       290                 295                 300
   Phe Gly Ile Ala Ile Val Val Phe Met Asn His Tyr Pro Leu Glu Lys
   305                 310                 315                 320
   Ile Gly Asp Pro Val Trp Asp Gly His Gly Phe Ser Val Gly Gln Ile
                   325                 330                 335
   His Glu Thr Met Asn Ile Arg Arg Gly Ile Ile Thr Asp Trp Phe Phe
               340                 345                 350
   Gly Gly Leu Asn Tyr Gln Ile Glu His His Leu Trp Pro Thr Leu Pro
               355                 360                 365
   Arg His Asn Leu Thr Ala Val Ser Tyr Gln Val Glu Gln Leu Cys Gln
       370                 375                 380
   Lys His Asn Leu Pro Tyr Arg Asn Pro Leu Pro His Glu Gly Leu Val
   385                 390                 395                 400
   Ile Leu Leu Arg Tyr Leu Ala Val Phe Ala Arg Met Ala Glu Lys Gln
                   405                 410                 415
   Pro Ala Gly Lys Ala Leu
               420
<210> 5
   <211> 27
   <212> DNA
   <213> Artificial Sequence
<220>
   <223> Description of Artificial Sequence: PCR Primer
<220>
   <221> variation
   <222> (1)..(27)
   <223> y = t or c
<220>
   <221> variation
   <222> (1)..(27)
   <223> n= a, t, c, or g
<220>
   <221> variation
   <222> (1)..(27)
   <223> r = a or g
<400> 5
   ggctggctga cncaygartt ytgycay 27
<210> 6
   <211> 30
   <212> DNA
   <213> Artificial Sequence
<220>
   <223> Description of Artificial Sequence: PCR Primer
<220>
   <221> variation
   <222> (1)..(30)
   <223> n = a, t, g, or c
<220>
   <221> variation
   <222> (1)..(30)
   <223> r = a or g
<220>
   <221> variation
   <222> (1)..(30)
   <223> y = t or c
<400> 6
   catcgttgga aanarrtgrt gytcdatytg 30
<210> 7
   <211> 41
   <212> DNA
   <213> Artificial Sequence
<220>
   <223> Description of Artificial Sequence: PCR Primer
<400> 7
   cccgggaagc ttctcgagga attttcaatc ctccttgggt c 41
<210> 8
   <211> 34
   <212> DNA
   <213> Artificial Sequence
<220>
   <223> Description of Artificial Sequence: PCR Primer
<400> 8
   cccgggtgga tccggaacat atcacacgaa acag 34
<210> 9
   <211> 6
   <212> RNA
   <213> Artificial Sequence
<220>
   <223> Description of Artificial Sequence: Polyadenylation Signal
<400> 9
   aauaaa 6
<210> 10
   <211> 20
   <212> DNA
   <213> Artificial Sequence
<220>
   <223> Description of Artificial Sequence: PCR Primer
<400> 10
   tctgggatct ctggttcttg 20
<210> 11
   <211> 9
   <212> RNA
   <213> Artificial Sequence
<220>
   <223> Description of Artificial Sequence: Histidine box
<400> 11
   uuuuuuucg 9
<210> 12
   <211> 5
   <212> PRT
   <213> Artificial Sequence
<220>
   <223> Description of Artificial Sequence: Histidine Box
<220>
   <221> VARIANT
   <222> (1)..(5)
   <223> Xaa = any amino acid
<400> 12
   His Xaa Xaa His His
    1               5
<210> 13
   <211> 5
   <212> PRT
   <213> Artificial Sequence
<220>
   <223> Description of Artificial Sequence: Histidine Box
<220>
   <221> VARIANT
   <222> (1)..(5)
   <223> Xaa = any amino acid
<400> 13
   Gln Xaa Xaa His His
    1               5

## Description

### FIELD OF THE INVENTION

The invention relates to desaturase enzymes that can be used to produce polyunsaturated fatty acids with important dietary applications.

### BACKGROUND

Fatty acids are fundamental components of living systems. They make up the major component of cytoplasmic membranes, common to plants, animals and protists alike.

Fatty acids of 20 carbons, with more than one unsaturated carbon-carbon bond along the hydrocarbon chain, are known to be of particular importance. Arachidonate (20:4) (Heinz, *Lipid Metabolism in Plants,* pp. 33-89, 1993; Yamazaki et al. *Biochim. Biophys. Acta* **1123**:18-26, 1992; Ulsamer et al., *J. Cell Biol.* **43**:105-114, 1969; and Albert et al. *Lipids* 14:498-500, 1979) and eicosapentaenoate (20:5) (Heinz, *Lipid Metabolism in Plants,* pp. 33-89, 1993; Yamazaki et al., *Biochim. Biophys. Acta* **1123**:18-26, 1992; Ulsamer et al., *J*. *Cell Biol.* **43**:105-114, 1969; Albert et al. *Lipids* **14**:498-500, 1979; and Cook et al., *J*. *Lipid Res.* **32**:1265-1273, 1991), commonly referred to as EPA, are significant components of mammalian cell membranes and are also precursors of signal molecules including prostaglandins. Certain specialized mammalian tissues such as brain (Naughton, *J. Biochem.* **13**:21-32, 1981), testes (Wilder and Coniglio, *Proc. Soc. Exp. Biol. Med.* **177**:399-405, 1984), and retina (Aveldano de Caldironi et al., *Prog. Lipid Res.* **20**:49-57, 1981) are especially rich in unsaturated fatty acids.

Arachidonate and eicosapentaenoate serve both as precursors for synthesis of 22-carbon polyunsaturated fatty acids and, with dihomo-γ-linoleate (20:3) (Yamazaki et al., *Biochim. Biophys. Acta* **1123**:18-26, 1992; Ulsamer et al., *J. Cell Biol.* **43**:105-114, 1969; and Albert et al., *Lipids* **14**:498-500, 1979), as precursors to the synthesis of eicosanoid metabolic regulators (Hwang, *Fatty Acids in Foods and Their Health Implications,* 545-557, 1992). Key enzymes in the synthesis of 20-carbon fatty acids are desaturases, which introduce cis double bonds by removing two hydrogen atoms at specific locations along the aliphatic hydrocarbon chains. Desaturase enzymes are specific to the position, number, and stereochemistry of the double bonds already present in the target fatty acid (Heinz, *Lipid Metabolism in Plants,* 33-89, 1993).

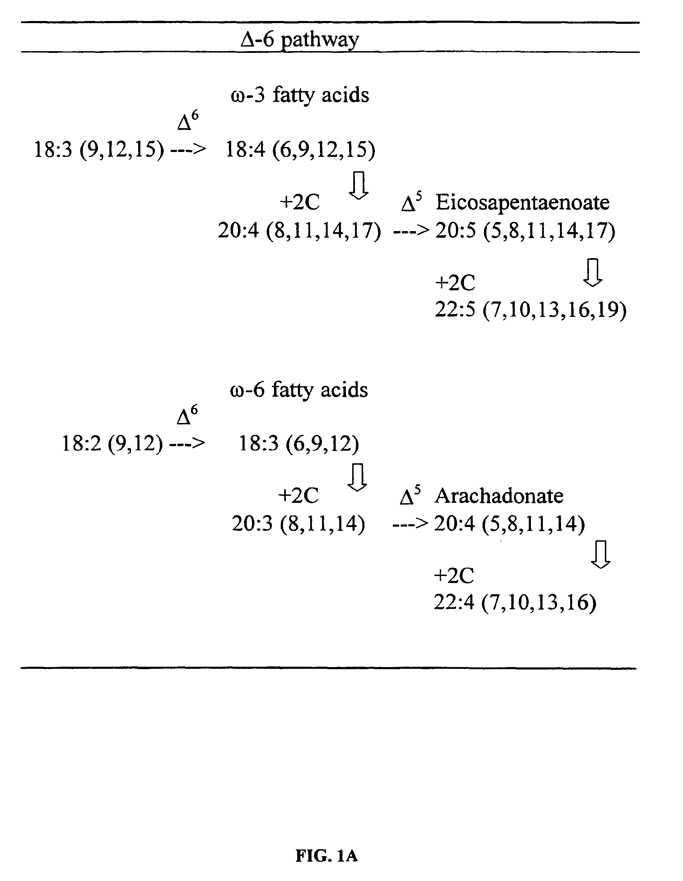

To synthesize 20-carbon polyunsaturated fatty acids, mammals must acquire the essential fatty acids 18:2 (Brenner, *The Role of Fats in Human Nutrition,* pp. 45-79, 1989) and 18:3 (Nelson, *Fatty Acids in Foods and Their Health Implications,* pp. 437-471, 1992; Brenner, *The Role of Fats in Human Nutrition,* pp. 45-79, 1989; and Hulanicka et al. *J. Biol. Chem.* **239:**2778-2787, 1964) from their diet (Nelson, *Fatty Acids in Foods and Their Health Implications,* 437-471,1992). These dietary polyunsaturated fatty acids are metabolized in the endoplasmic reticulum by an alternating series of position-specific desaturases and malonyl-CoA-dependent chain-elongation steps (FIG. 1A), which results in the characteristic methylene-interrupted double bond pattern. In the liver, which is the primary organ of human lipid metabolism, the first step in biosynthesis of 20-carbon fatty acids is desaturation of the essential fatty acids at the Δ⁶ position. The desaturation products are elongated to 20:3 and 20:4 (Cook et al., *J*. *Lipid Res.* **32**:1265-1273, 1991). In turn, these 20-carbon products are desaturated by a Δ⁵-desaturase to produce arachidonate and eicosapentaenoate. The Δ⁶-desaturation step is rate-limiting in this metabolic pathway (Bernet and Sprecher, *Biochim. Biophys. Acta* **398**:354-363, 1975; and Yamazaki et al., *Biochim. Biophys. Acta* **1123:**18-26, 1992) and, not surprisingly, is subject to regulation by dietary and hormonal changes (Brenner, *The Role* of *Fats in Human Nutrition,* pp. 45-79, 1989).

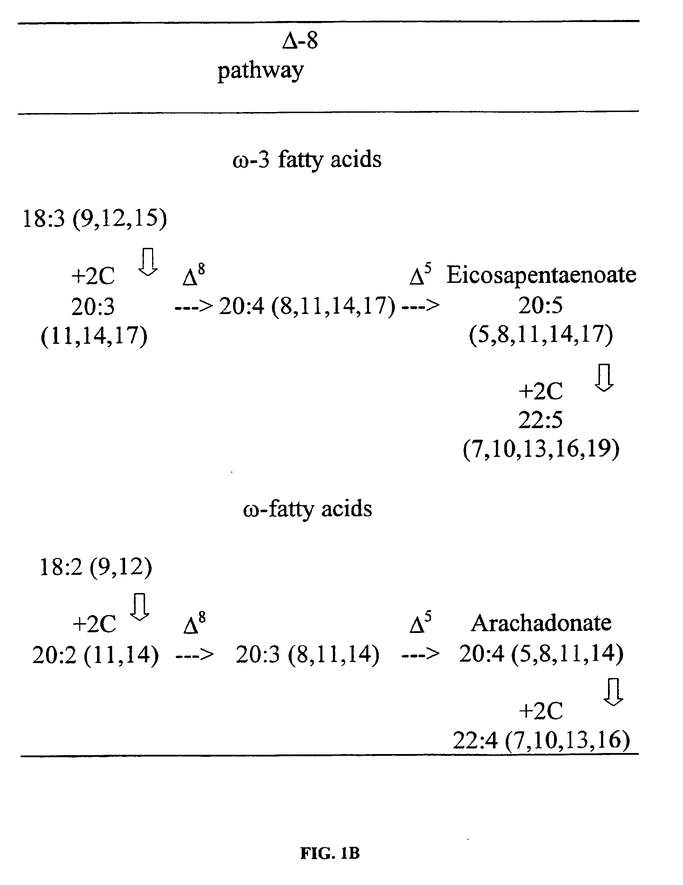

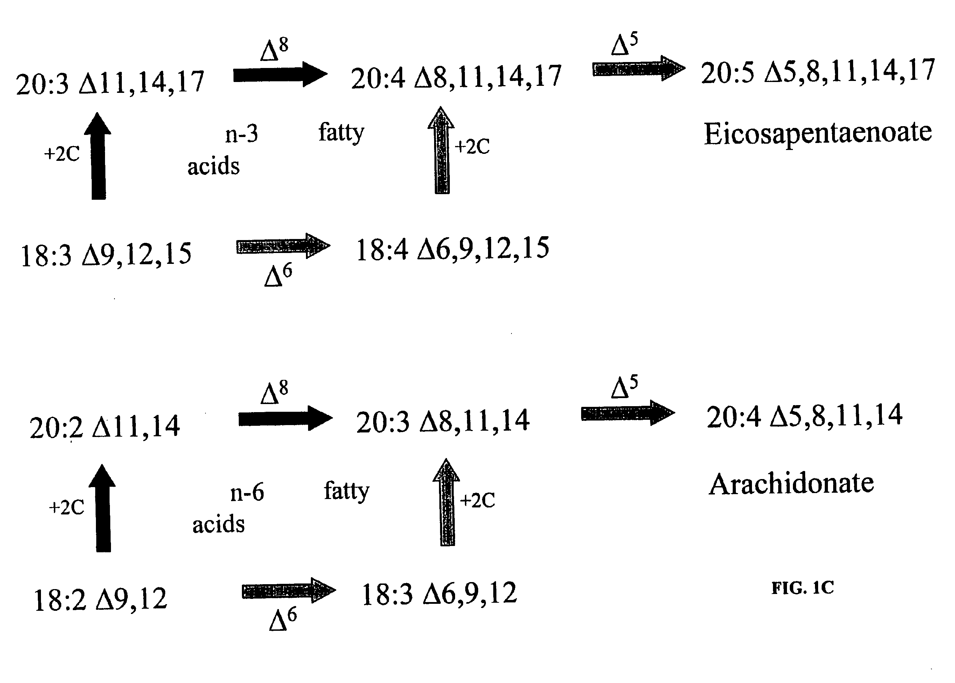

In contrast to the liver, an alternate pathway for biosynthesis of 20-carbon polyunsaturated fatty acids has been demonstrated in a few organisms and tissues (FIG. 1B). Instead of desaturation, the first step in the alternate pathway is elongation of the essential 18-carbon fatty acids to 20-carbon chain lengths, producing 20:2 (Ulsamer et al., *J. Cell Biol.* **43:**105-114, 1969; and Albert et al. *Lipids* **14:**498-500, 1979) and 20:3. Subsequent desaturation occurs via a Δ⁸ -desaturase (FIG. 1). The products of this elongation-desaturation, 20:3 and 20:4, are the same as the more usual desaturation-elongation pathway. The Δ⁸ pathway is present in the soil amoebae *Acanthamoeba sp.* (Ulsamer et al, *J. Cell Biol.* **43:**105-114, 1969), and in euglenoid species, where it is the dominant pathway for formation of 20-carbon polyunsaturated fatty acids (Hulanicka et al., *Journal of Biological Chemistry* **239**:2778-2787, 1964).

This Δ⁸-desaturation pathway occurs in mammals, both in rat testis (Albert and Coniglio, *Biochim. Biophys. Acta* **489**:390-396, 1977) and in human testis (Albert et al., *Lipids* **14**:498-500, 1979). While Δ⁸ activity has been observed in breast cancer cell lines (Grammatikos et al., *Br. J. Cancer* **70**:219-227, 1994; and Bardon et al., *Cancer Lett.* **99**:51-58, 1996) and in glioma (Cook et al., *J. Lipid* Res. **32**:1265-1273, 1991), no Δ⁸ activity is detectable in a corresponding non-cancerous breast cell line (Grammatikos et al., *Br. J.* Cancer **70**:219-227, 1994) or in the brain (Dhopeshwarkar and Subramanian, *J. Neurochem.* **36**:1175-1179, 1976). The significance of Δ⁸-desaturation to normal or cancer cell metabolism is unclear, since analysis of desaturase activities in mammalian systems is frequently complicated by the presence of competing Δ⁶ reactions and chain-shortening retroconversion of fatty acid substrates in tissue (Sprecher and Lee, *Biochim. Biophys. Acta* **388**:113-125, 1975; Geiger et al., *Biochim. Biophys. Acta* **1170**:137-142, 1993).

Polyunsaturated 20-carbon fatty acids are, for the reasons outlined above, important in the human diet, and there has been considerable recent interest in incorporating such fatty acids into infant food, baby formula, dietary supplements, and nutriceutical formulations.

It would therefore be desirable to produce new transgenic plants and animals with enhanced ability to produce polyunsaturated 20-carbon fatty acids. Spychalla et al, PNAS, 94, p1142-1147, 1997, and Sagarova et al, PNAS, 94, p 4211-4216, 1997, both relate to closing and characterising a desaturase encoding gene.

### SUMMARY OF THE DISCLOSURE

The invention provides novel desaturase enzymes as claimed that may be cloned and expressed in the cells of various organisms, including plants to produce 20-carbon polyunsaturated fatty acids. Expression of such fatty acids enhances the nutritional qualities of such organisms. For instance, oil-seed plants may be engineered to incorporate the Δ⁵ - and Δ⁸ desaturases of the invention. Such oil-seed plants would produce seed-oil rich in polyunsaturated 20:3, 20:4, 20:5, 22:4, and 22:5 fatty acids. Such fatty acids could be incorporated usefully into infant-formula, foods of all kinds, dietary supplements, nutriceutical, and pharmaceutical formulations.

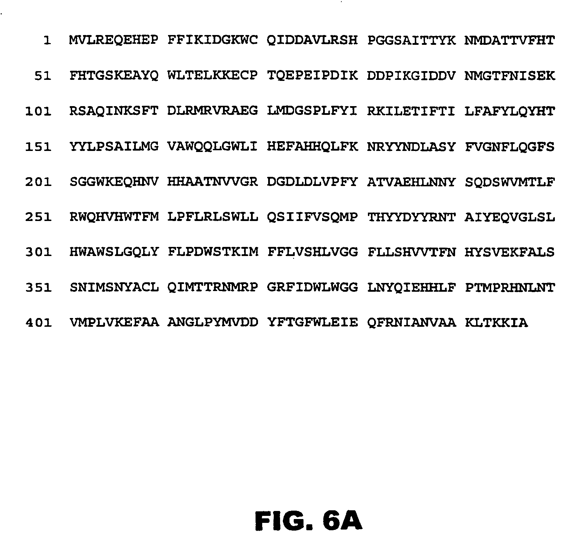

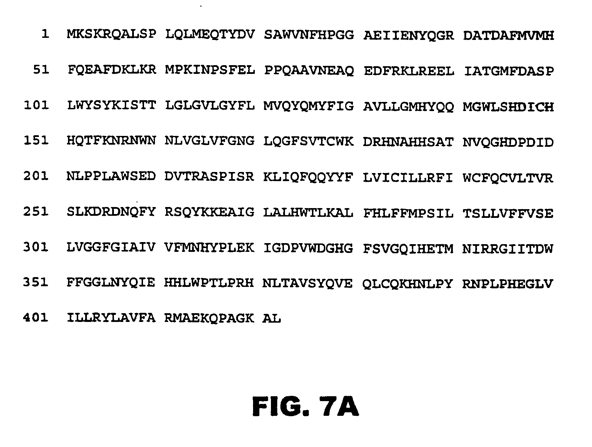

The invention also provides claimed proteins differing from the proteins of FIG. 6A and FIG. 7A by one or more conservative amino acid substitutions. Also provided are proteins that exhibit "substantial similarity" (defined in the "Definitions section) With the proteins of FIG. 6A and FIG. 7A.

The invention provides claimed isolated novel nucleic acids that encode the above-mentioned proteins, recombinant nucleic acids that include such nucleic acids and cells, and plants and organisms containing such recombinant nucleic acids.

The novel desaturase enzymes can be used individually, or in conjunction with one another, for instance in a metabolic pathway, to produce polyunsaturated fatty acid, such as 20.3, 20.4, 20.5, 22.4, and 22.5 fatty acids.

The scope of the invention also includes portions of nucleic acids encoding the novel enzymes portions of nucleic acids that encode polypeptides substantially similar to these novel enzymes, and portions of nucleic acids that encode polypeptides that differ from the proteins of FIG. 6A and FIG. 7A by one or more conservative amino acid substitutions. Such portions of nucleic acids may be used, for instance, as primers and probes for research and diagnostic purposes. Research applications for such probes and primers include the identification and cloning of related Δ⁵-and Δ⁸-desaturases in other organisms including humans.

The invention also includes methods that utilize the desaturase enzymes of the invention. An example of this embodiments is a yeast or plant cell that carries genes for one or both desaturases of the invention and that, by virtue of these desaturases, is able to produce arachidonic acid and/or EPA.

### BRIEF DESCRIPTION OF THE DRAWINGS

FIG. 1A shows a common pathway for synthesis of 20-carbon polyunsaturated fatty acids that begins with Δ⁶ desaturation of 18-carbon fatty acids followed by 2-carbon elongation, and then further desaturation and elongation.
FIG. 1 B shows an alternate pathway that begins with an elongation of 18-carbon fatty acid to 20-carbon fatty acids, followed by Δ⁸ desaturation and a second desaturation at the Δ⁵ position.
FIG. 1C shows alternate pathways for the synthesis of polyunsaturated fatty acids using Δ⁵-, Δ⁶-, and Δ⁸ -desaturases to produce arachidonic acid and EPA.

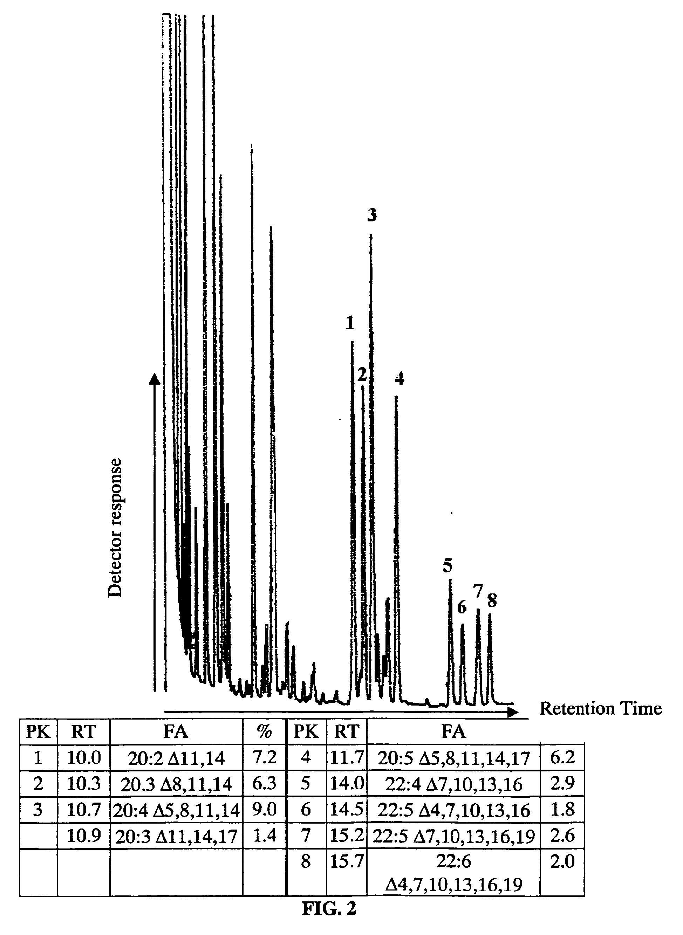

FIG. 2 shows gas chromatographic (GC) analysis of fatty acid methyl esters from *E*. *gracilis* grown (heterotrophically) in the dark with sucrose as carbon source. Fatty acids were identified by comparison of retention times with known standards. Significant peaks are numbered with their retention times and proportion of the total fatty acid indicated.

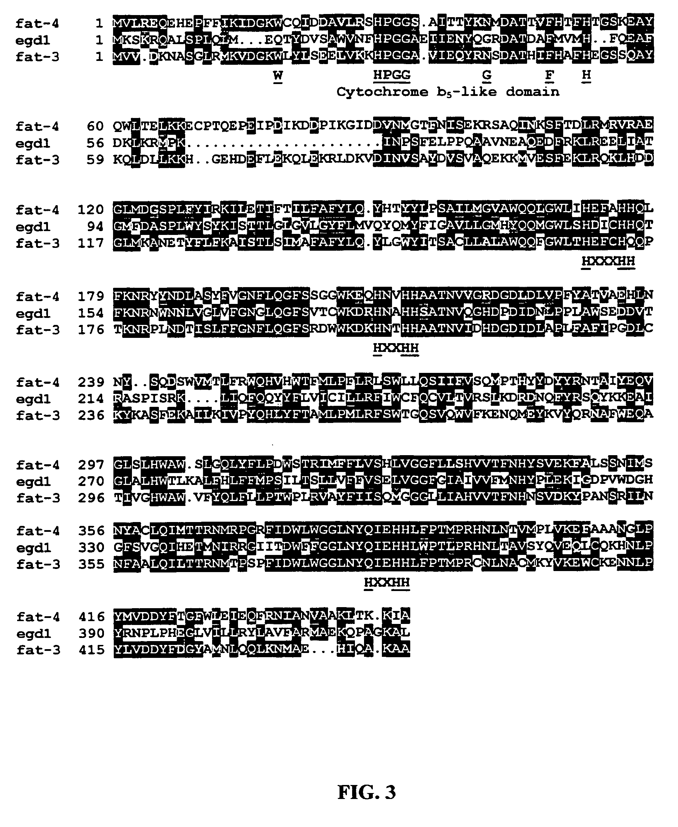

FIG. 3 shows amino acid sequence similarities between the *Euglena* Δ⁸-desaturase protein (EFD1) and the desaturase enzymes of *C. elegans.* The deduced amino acid sequence of the *EFD1* gene shows similarity with the *C. elegans* Δ⁶ (FAT-3) and Δ⁵ (FAT-4) desaturases (Napier et al., *Biochem. J*. **330**:611-614, 1998). The similarity is strongest in the regions of conserved function. In the N-terminal region amino acids forming a cytochrome b₅ -like domain (Lederer, *Biochimie* **76**:674-692, 1994) are indicated. The His-box motifs indicated by underlined characters are present in other identified membrane desaturases (Napier et al., *Biochem. J*. **330**:611-614, 1998; Michaelson et al., *J*. *BioL Chem.* **273:**19055-19059, 1998; and Shanklin and Cahoon, *Annu. Rev. Plant Physiol. Plant Mol. Biol.* **48**:611-641, 1998).

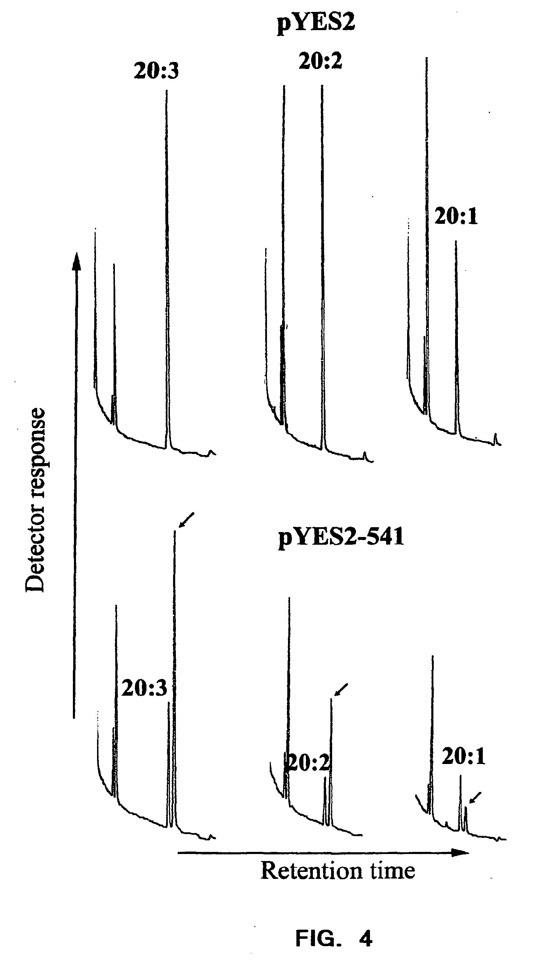

FIG. 4 shows the results of gas chromatography of fatty acid methyl esters from recombinant yeast. Cultures of yeast containing either control pYES2 or pYES2-541, which expresses the *Euglena* Δ⁸-desaturase (*EFD1*) gene, were supplemented with the indicated 20-carbon fatty acids. The control strain does not desaturate the exogenous fatty acids. For the experimental strain, an arrow indicates the desaturation peak.

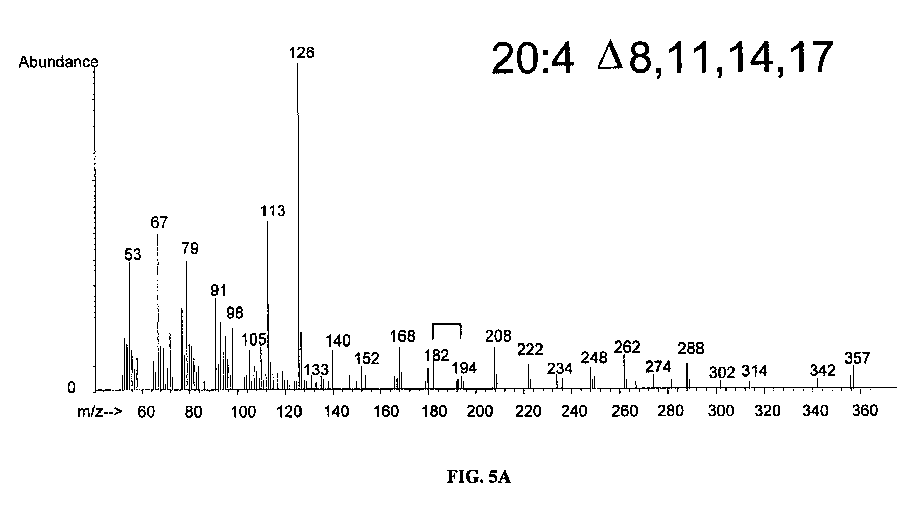

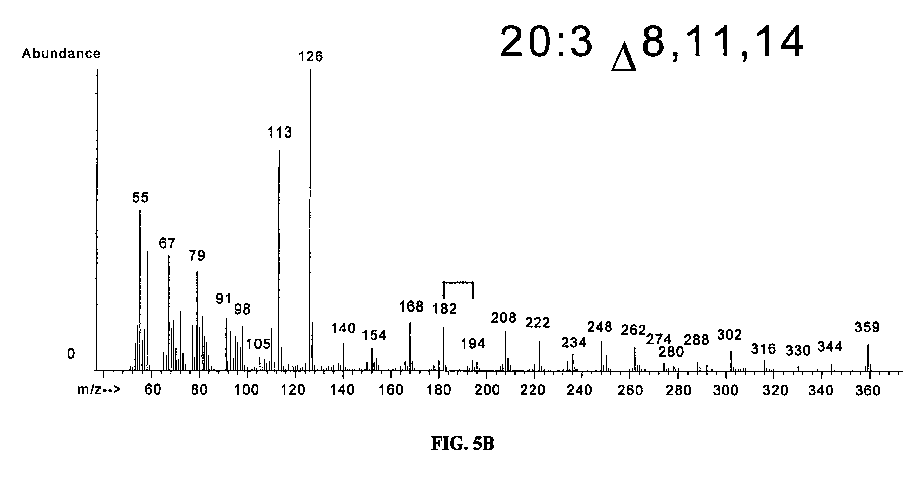

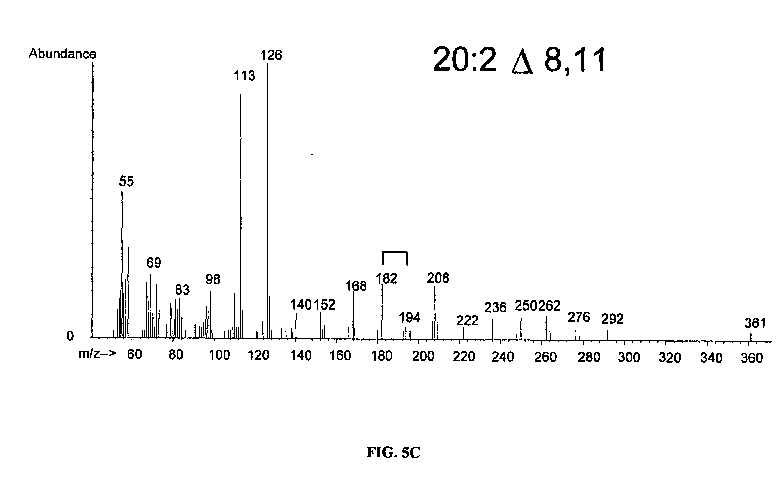

FIG. 5 shows the results of mass spectrometry (MS) of desaturation products. DMOX derivatives of EFD1 desaturation products were analyzed by GC-mass spectrometry. The molecular ion of each fatty acid is 2 a.m.u. (atomic mass units) less than the substrate provided, as expected for insertion of a double bond. Desaturation at the Δ⁸ position is established by characteristic m/z peaks of 182 and 194 for each product, indicated by the bracket.
FIG. 6A shows the primary amino acid sequence of the fatty acid Δ⁵-desaturase from *Caenorhabditis elegans.*

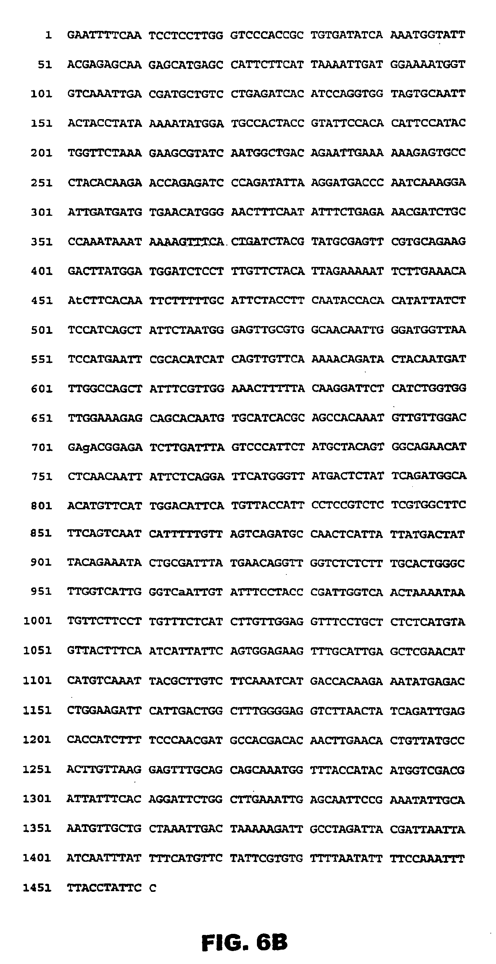

FIG. 6B shows a nucleotide sequence including the ORF (open reading frame) that encodes the fatty acid Δ⁵-desaturase from *Caenorhabditis elegans.*
FIG. 7A shows the primary amino acid sequence of the fatty acid Δ⁸-desaturase from the protist *Euglena gracilis.*

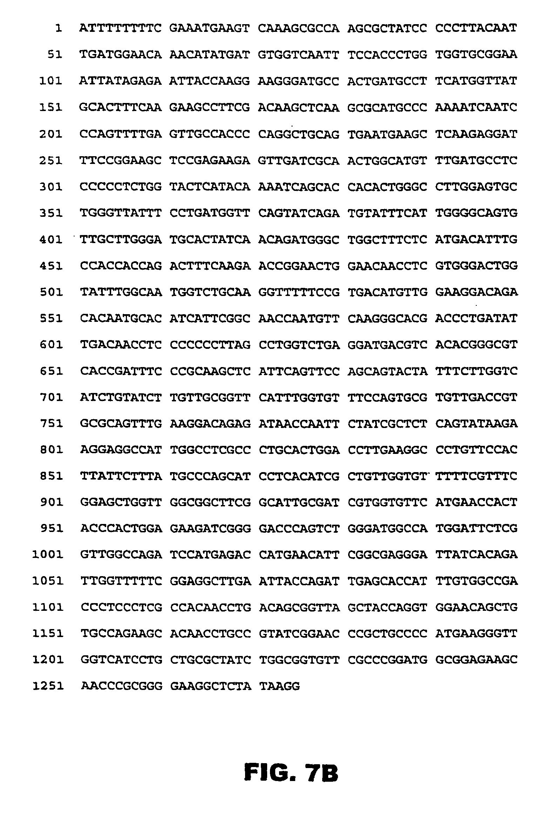

FIG. 7B shows a nucleotide sequence including the ORF that encodes the Δ⁸-desaturase from the protist *Euglena gracilis.*

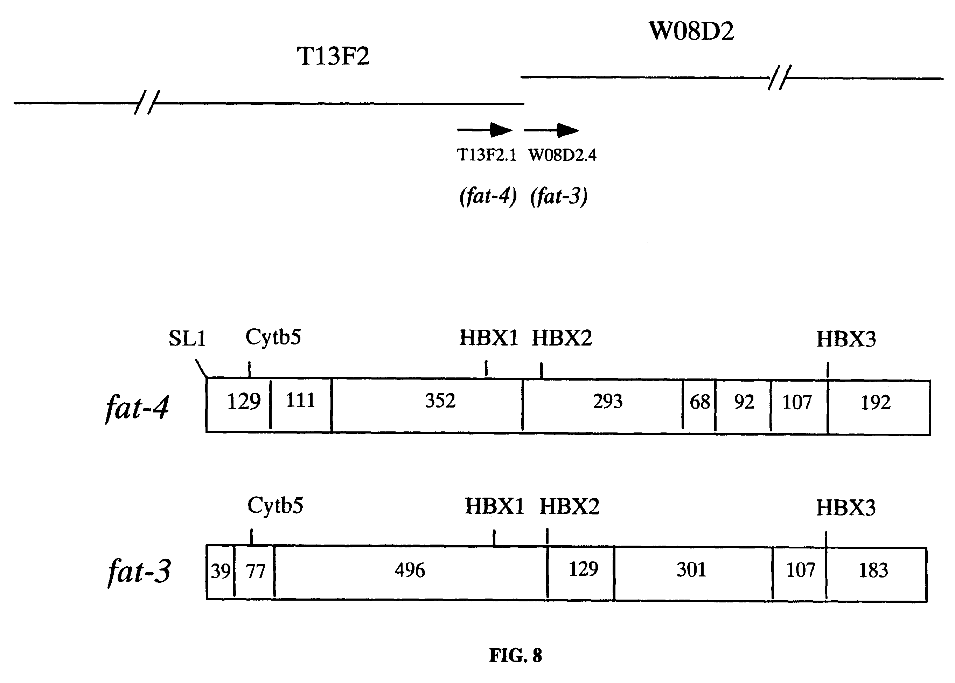

FIG. 8 shows certain features of the structure of the C. *elegans* Δ⁵ - and Δ⁶-desaturase genes. The relative location of gene products T13F2.1 and W08D2.4 on their respective cosmids is shown above. The exon structure of T13F2.1 (*fat-4*) and W08D2.4 *(fat-3)* showing the sites of sequences encoding the SL1 splice site, the heme-binding motif of cytochrome b₅ (cyt b₅), and the three conserved histidine box motifs (HBX) is shown below.

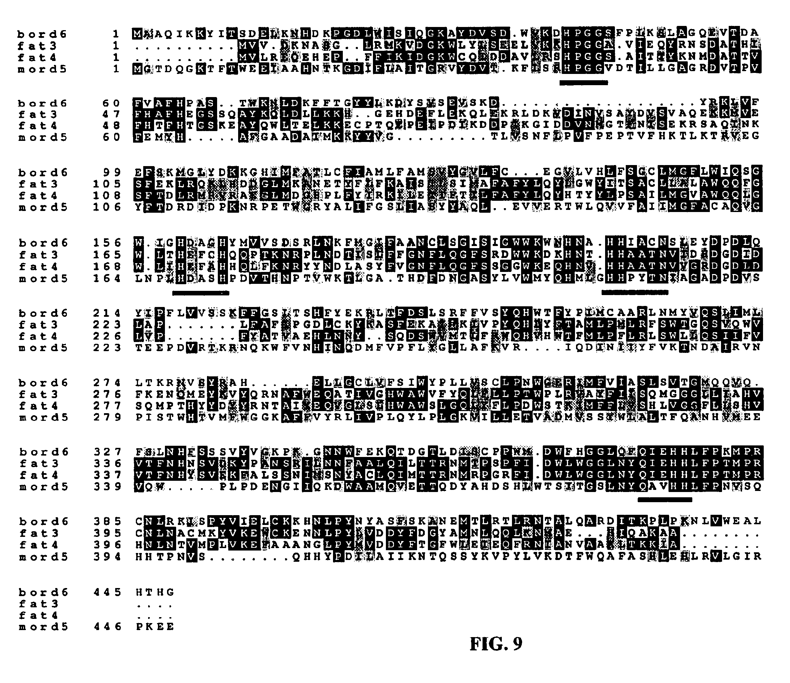

FIG. 9 shows a comparison of the predicted amino acid sequences of the borage Δ⁶-desaturase (bord6), *C. elegans* FAT-3 (fat3), *C. elegans* FAT-4 (fat4), and the *Mortierella alpina* Δ⁵-(mord5) desaturase. Identical or conserved residues are shaded, and the conserved HPGG heme-binding domain and the conserved histidine boxes are underlined. Abbreviations: bord6 = *Borago officinalis* Δ⁶-desaturase (GenBank accession number U79010); fat4 = C. *elegans* FAT-4 desaturase; fat3 = C. *elegans* Δ⁶ desaturase sequence of W08D2.4 (GenBank accession number Z70271), edited to remove amino acids 38-67, on the basis of the cDNA sequence; mord5 = *Mortierella alpina* Δ⁵ desaturase (GenBank accession number AF054824).

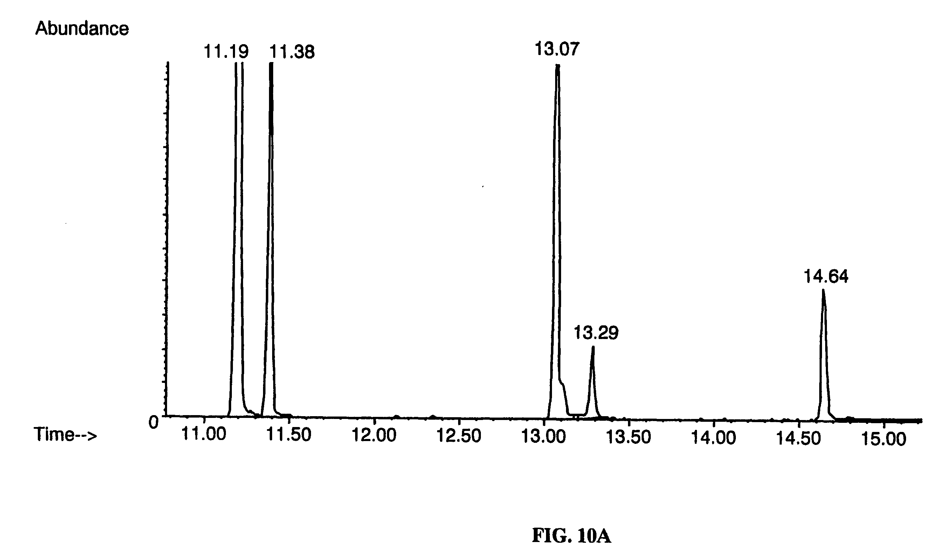

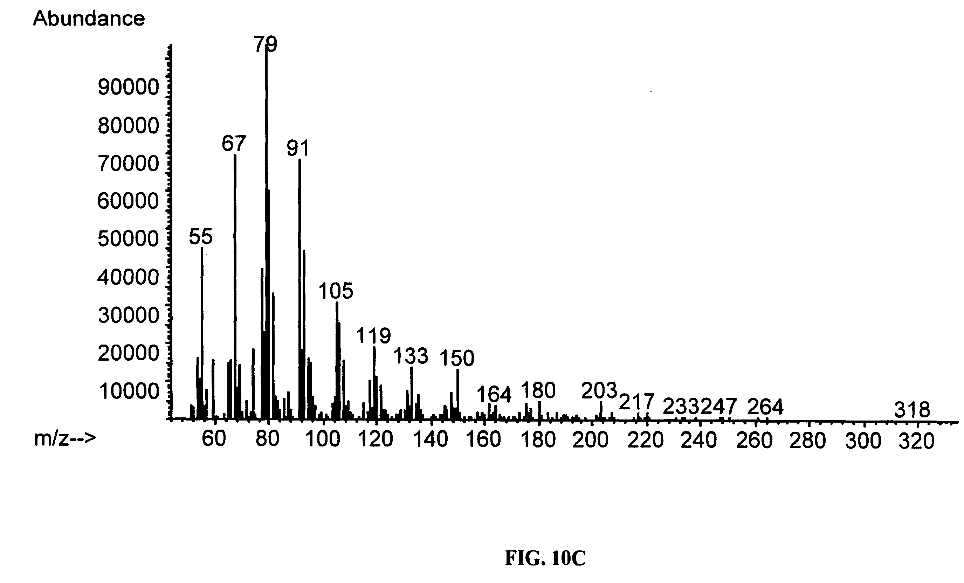

FIGS. 10A-10C show identification of arachidonic acid in transgenic yeast by gas chromatography-mass spectroscopy (GC-MS). Fatty acid methyl esters of total lipids of S. *cerevisiae* grown for 16 hours under inducing conditions (2% galactose) supplemented with 0.2 mM di-homo-γ-linolenic acid were analyzed by GC-MS. (A) Yeast transformed with (empty) vector pYES2. (B) Yeast transformed with pYES2 vector carrying *fat-4.* The common peaks were identified as 16:0 (11.19-11.12 min.), 16:1 (11.38 min.), 18:0 (13.07-13.08 min.), 18:1 (13.29 min.), 20:3 (11.64-11.65 min.). The novel peaks are arachidonic acid (14.49 min.) and 18:2 (12.91 min.). (C) The mass spectrum of the peak eluting at 14.49 min. This spectrum is indistinguishable from that of authentic methyl-arachidonate.

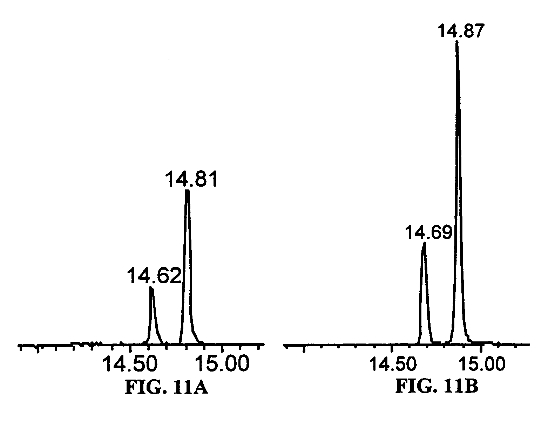

FIGS. 11A and 11 B show the novel desaturation products from substrates lacking a Δ⁸ double bond. (A) Partial GC trace of fatty acid methyl esters derived from yeast expressing the *fat-4* Δ⁵-desaturase supplemented with 20:20Δ^{11,14} (14.81 min.). The desaturation product of this substrate elutes at 14.62 min. and has been identified as 20:3Δ^{5,11,14} . (B) Partial GC trace of yeast expressing the *fat-4* Δ⁵-desaturase supplemented with 20:3Δ^{11,14,17} (14.87 min.). The desaturation product of this substrate elutes at 14.69 min. and has been identified as 20:4Δ^{5,11,14,17}.

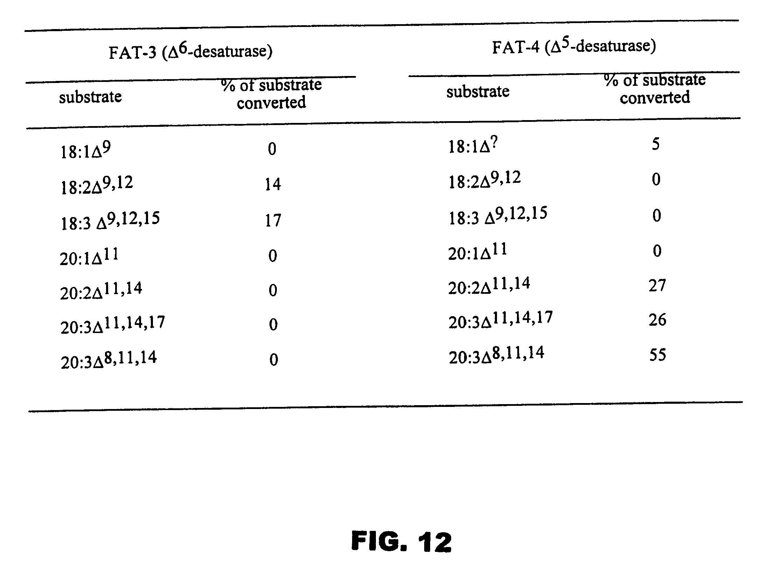

FIG. 12 is a table comparing the substrate specificities of *C*. *elegans* Δ⁵ - and Δ⁶-desaturases.

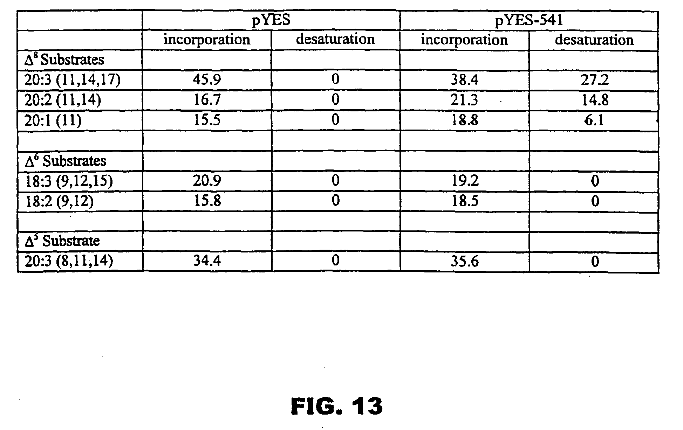

FIG. 13 is a table comparing incorporation and desaturation of fatty acids by yeast strains transformed with a control construct pYES, and with pYES-541, a clone containing EGD1, the *E. gracilis* Δ⁸-desaturase gene. *S. cervisiae* strains containing a control vector (pYES) or expressing EFD1 (pYES-541) were cultured in the presence of the indicated fatty acids. The cultures were harvested, washed, and methyl esters prepared from total cells and analyzed by GC. The weight % of total fatty acid methyl esters is indicated.

### SEQUENCE LISTING

The nucleic and amino acid sequences listed in the accompanying sequence listing are shown using standard letter abbreviations for nucleotide bases, and three-letter code for amino acids. Only one strand of each nucleic acid sequence is shown, but the complementary strand is understood to be included by any reference to the displayed strand.

SEQ ID NO: 1 is the nucleotide sequence corresponding to the open reading frame of the fatty acid Δ⁵-desaturase from *Caenorhabditis elegans.*

SEQ ID NO: 2 is the primary amino acid sequence of the fatty acid Δ⁵-desaturase from *Caenorhabditis elegans.*

SEQ ID NO: 3 is the nucleotide sequence corresponding to the open reading frame of the fatty acid Δ⁸-desaturase from the protist *Euglena gracilis.*

SEQ ID NO: 4 is the primary amino acid sequence of fatty acid Δ⁸-desaturase from the protist *Euglena gracilis.*

SEQ ID NOs: 5-8 are primers used to amplify and clone the Δ⁸-desaturase-encoding nucleic acid sequence.

SEQ ID NO: 9 is a polyadenylation signal.

SEQ ID NO: 10 is a primer used to amplify and clone the Δ⁵-desaturase-encoding nucleic acid sequence.

SEQ ID NO: 11 is a short RNA leader sequence.

SEQ ID NO: 12 is the amino acid sequence of a histidine box motif.

SEQ ID NO: 13 is the amino acid sequence of a histidine box motif

### DESCRIPTION OF THE INVENTION

The following definitions and methods are provided to better define the present invention and to guide those of ordinary skill in the art in the practice of the present invention. Unless otherwise noted, terms are to be understood according to conventional usage by those of ordinary skill in the relevant art. Definitions of common terms in molecular biology may also be found in Rieger *et al., Glossary of Genetics: Classical and Molecular,* 5th edition, Springer-Verlag: New York, 1991; and Lewin, *Genes VI,* Oxford University Press: New York, 1997. The nomenclature for DNA bases as set forth at 37 C.F.R. § 1.822 is used. The standard one- and three-letter nomenclature for amino acid residues is used.

### Definitions

**Portion:** A portion of a nucleic acid molecule is a stretch of contiguous nucleic acids corresponding to the sequence of that molecule that may be about 15, 20, 30, 40, 50, or 60 nucleic acids in length. Such nucleotide portions may be used as probes or primers. A portion of a protein is a stretch of contiguous amino acids corresponding to the amino acid sequence of that protein that may be about 5, 10, 20, 30, 40, or 50 residues in length. As used herein, such a portion may correspond to any segment of a nucleic acid molecule, for instance such a portion may correspond to a segment consisting of nucleotides 1-500, 501-1000, or 1001-1451 of the sequence shown in FIG. 6B, or nucleotides 1-400, 401-800, 801-1251 of the sequence shown in FIG. 7B.

**Desaturase:** A desaturase is an enzyme that promotes the formation of carbon-carbon double bonds in a hydrocarbon molecule.

Desaturase activity may be demonstrated by assays in which a preparation containing an enzyme is incubated with a suitable form of substrate fatty acid and analyzed for conversion of this substrate to the predicted fatty acid product. Alternatively, a DNA sequence proposed to encode a desaturase protein may be incorporated into a suitable vector construct and thereby expressed in cells of a type that do not normally have an ability to desaturate a particular fatty acid substrate. Activity of the desaturase enzyme encoded by the DNA sequence can then be demonstrated by supplying a suitable form of substrate fatty acid to cells transformed with a vector containing the desaturase-encoding DNA sequence and to suitable control cells (for example, transformed with the empty vector alone). In such an experiment, detection of the predicted fatty acid product in cells containing the desaturase-encoding DNA sequence and not in control cells establishes the desaturase activity. Examples of this type of assay have been described in, for example, Lee et al., *Science* **280**:915-918, 1998; Napier et al., *Biochem. J*. **330:**611-614, 1998; and Michaelson et al., *J*. *Biol. Chem.* **273**:19055-19059, 1998, which are incorporated herein by reference.

The Δ⁵ -desaturase activity may be assayed by these techniques using, for example, 20:3Δ^{8,11,14} as substrate and detecting 20:4Δ^{5,8,11,14} as the product (Michaelson et al., *J*. *Biol.* Chem. 273:19005-19059, 1998). Other potential substrates for use in Δ⁵ activity assays include (but are not limited to) 10:2Δ^{11,14} (yielding 20:5Δ^{5,11,14} as the product) and 20:3Δ^{11,14,12} (yielding 20:4Δ^{5,11,14,17} as the product).

The Δ⁸ -desaturase may be assayed by similar techniques using, for example, 20:3Δ^{11,14,17} as the substrate and detecting 20:4Δ^{8,11,14,17} as the product.

**ORF:** Open reading frame. An ORF is a contiguous series of nucleotide triplets coding for amino acids. These sequences are usually translatable into a peptide.

**Homologs:** Two nucleotide or amino acid sequences that share a common ancestral sequence and diverged when a species carrying that ancestral sequence split into two species. Homologs frequently show a substantial degree of sequence identity.

**Transformed:** A transformed cell is a cell into which has been introduced a nucleic acid molecule by molecular biology techniques. The term encompasses all techniques by which a nucleic acid molecule might be introduced into such a cell, including transfection with viral vectors, transformation with plasmid vectors, and introduction of naked DNA by electroporation, lipofection, and particle gun acceleration.

**Purified:** The term purified does not require absolute purity; rather, it is intended as a relative term. Thus, for example, a purified protein preparation is one in which the subject protein or other substance is more pure than in its natural environment within a cell. Generally, a protein preparation is purified such that the protein represents at least 50% of the total protein content of the preparation.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame. If introns are present, the operably linked DNA sequences may not be contiguous.

**Cell**: A plant, animal, protist, bacterial, or fungal cell.

**Sequence similarity:** The similarity between two nucleic acids or two amino acid sequences is expressed in terms of percentage sequence identity. The higher the percentage sequence identity between two sequences, the more similar the two sequences are.

In the case of protein alignments, similarity is measured not only in terms of percentage identity, but also takes into account conservative amino acid substitutions. Such conservative substitutions generally preserve the hydrophobicity and acidity of the substituted residue, thus preserving the structure (and therefore function) of the folded protein. The computer programs used to calculate protein similarity employ standardized algorithms that, when used with standardized settings, allow the meaningful comparison of similarities between different pairs of proteins.

Sequences are aligned, with allowances for gaps in alignment, and regions of identity are quantified using a computerized algorithm. Default parameters of the computer program are commonly used to set gap allowances and other variables.

Methods of alignment of sequences for comparison are well-known in the art. Various programs and alignment algorithms are described by Pearson et al., *Methods in Molecular Biology* **24**: 307-331, 1994, and in Altschul et al., *Nature Genet.* **6**:119-129, 1994. Altschul et al. presents a detailed consideration of sequence alignment methods and homology calculations.

The NCBI Basic Local Alignment Search Tool (BLAST^{™}) (Altschul et al., *J*. *Mol. Biol.* **215**:403-410, 1990 is available from several sources, including the National Center for Biotechnology Information (NBCI, Bethesda, MD) and on the Internet, for use in connection with the sequence analysis programs blastp, blastn, blastx, tblastn and tblastx. BLAST^{™} can be accessed at htp://www.ncbi.nlm.nih.gov/BLAST/. A description of how to determine sequence identity using this program is available at the web site. As used herein, sequence identity is commonly determined with the BLAST^{™} software set to default parameters. For instance, blastn (version 2.0) software may be used to determine sequence identity between two nucleic acid sequences using default parameters (expect = 10, matrix = BLOSUM62, filter = DUST (Tatusov and Lipmann, in preparation as of December 1, 1999; and Hancock and Armstrong, *Comput. Appl. Biosci.* **10:**67-70, 1994), gap existence cost = 11, per residue gap cost = 1, and lambda ratio = 0.85). For comparison of two polypeptides, blastp (version 2.0) software may be used with default parameters (expect 10, filter = SEG (Wootton and Federhen, *Computers in Chemistry* **17**:149-163, 1993), matrix = BLOSUM62, gap existence cost = 11, per residue gap cost = 1, lambda = 0.85).

When aligning short peptides (fewer than around 30 amino acids), the alignment should be performed using the Blast 2 sequences function, employing the PAM30 matrix set to default parameters (open gap 9, extension gap 1 penalties).

An alternative alignment tool is the ALIGN Global Optimal Alignment tool (version 3.0) available from Biology Workbench at http://biology.ncsa.uiuc.edu. This tool may be used with settings set to default parameters to align two known sequences. References for this tool include Meyers and Miller, *CABIOS* **4**:11-17, 1989.

Conservative amino acid substitutions are those substitutions that, when made, least interfere with the properties of the original protein, i.e., the structure and especially the function of the protein is conserved and not significantly changed by such substitutions. The table below shows amino acids that may be substituted for an original amino acid in a protein and that are regarded as conservative substitutions.

**TABLE 1**

| Original Residue | Conservative Substitutions |
|---|---|
| ala | ser |
| arg | lys |
| asn | gln; his |
| asp | glu |
| cys | ser |
| gln | asn |
| glu | asp |
| gly | pro |
| his | asn; gln |
| ile | leu; val |
| leu | ile; val |
| lys | arg; gln; glu |
| met | leu; ile |
| phe | met; leu; tyr |
| ser | thr |
| thr | ser |
| trp | tyr |
| tyr | trp; phe |
| val | ile; leu |

Conservative substitutions generally maintain (a) the structure of the polypeptide backbone in the area of the substitution, for example, as a sheet or helical conformation, (b) the charge or hydrophobicity of the molecule at the target site, or (c) the bulk of the side chain.

The substitutions which in general are expected to produce the greatest changes in protein properties will be non-conservative, for instance changes in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histadyl, is substituted for (or by) an electronegative residue, e.g., glutamyl or aspartyl; or (d) a residue having a bulky side chain, e.g., phenylalanine, is substituted for (or by) one not having a side chain, e.g., glycine.

**Probe:** An isolated nucleic acid attached to a detectable label or reporter molecule. Typical labels include radioactive isotopes, ligands, chemiluminescent agents, and enzymes.

**Primers:** Short nucleic acids, preferably DNA oligonucleotides 10 nucleotides or more in length, that are annealable to a complementary target DNA strand by nucleic acid hybridization to form a hybrid between the primer and the target DNA strand, then extendable along the target DNA strand by a DNA polymerase enzyme. Primer pairs can be used for amplification of a nucleic acid sequence, e.g., by the polymerase chain reaction (PCR) or other nucleic-acid amplification methods known in the art.

Probes and primers as used in the present invention typically comprise at least 15 contiguous nucleotides. In order to enhance specificity, longer probes and primers may also be employed, such as probes and primers that comprise at least 20, 30, 40, 50, 60, 70, 80, 90, 100, or 150 consecutive nucleotides of the disclosed nucleic acid sequences.

Alternatively, such probes and primers may comprise at least 15, 20, 30, 40, 50, 60, 70, 80, 90, 100, or 150 consecutive nucleotides that share a defined level of sequence identity with one of the disclosed sequences, for instance, at least a 50%, 60%, 70%, 80%, 90%, or 95% sequence identity.

Alternatively, such probes and primers may be nucleotide molecules that hybridize under specific conditions and remain hybridized under specific wash conditions such as those provided below. These conditions can be used to identifying variants of the desaturases. Nucleic acid molecules that are derived from the desaturase cDNA and gene sequences include molecules that hybridize under various conditions to the disclosed desaturase nucleic acid molecules, or fragments thereof. Generally, hybridization conditions are classified into categories, for example very high stringency, high stringency, and low stringency. The conditions for probes that are about 600 base pairs or more in length are provided below in three corresponding categories.

**Very High Stringency (detects sequences that share 90% sequence identity)**

| | | | | | |
|---|---|---|---|---|---|
| Hybridization | in | SSC | at | 65°C | 16 hours |
| Wash twice | in | SSC | at | room temp. | 15 minutes each |
| Wash twice | in | SSC | at | 65°C | 20 minutes each |

**High Stringency (detects sequences that share 80% sequence identity or greater)**

| | | | | | |
|---|---|---|---|---|---|
| Hybridization | in | SSC | at | 65°C-70°C | 16-20 hours |
| Wash twice | in | SSC | at | room temp. | 5-20 minutes each |
| Wash twice | in | SSC | at | 55°C-70°C | 30 minutes each |

**Low Stringency (detects sequences that share greater than 50% sequence identity)**

| | | | | | |
|---|---|---|---|---|---|
| Hybridization | in | SSC | at | room temp.-55°C | 16-20 hours |
| Wash at least twice | in | SSC | at | room temp.-55°C | 20-30 minutes each |

Methods for preparing and using probes and primers are described in the references, for example Sambrook et al. *Molecular Cloning: A Laboratory Manual,* 2^{nd} ed., Cold Spring Harbor Laboratory Press, NY, 1989; Ausubel et al. *Current Protocols in Molecular Biology,* Greene Publishing Associates and Wiley-Intersciences, 1987; and Innis et al., *PCR Protocols, A Guide to Methods and Applications,* Academic Press, Inc., San Diego, California. 1990. PCR primer pairs can be derived from a known sequence, for example, by using computer programs intended for that purpose such as Primer (Version 0.5, 1991, Whitehead Institute for Biomedical Research, Cambridge, MA).

**Recombinant nucleic acid:** A sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, e.g., by genetic engineering techniques such as those described in Sambrook et al. *Molecular Cloning: A Laboratory Manual,* 2^{nd} ed., Cold Spring Harbor Laboratory Press, NY, 1989. The term recombinant includes nucleic acids that have been altered solely by addition, substitution, or deletion of a portion of the nucleic acid.

**Native:** The term "native" refers to a naturally-occurring ("wild-type") nucleic acid or polypeptide. The native nucleic acid or protein may have been physically derived from a particular organism in which it is naturally occurring or may be a synthetically constructed nucleic acid or protein that is identical to the naturally-occurring nucleic acid or protein.

**Isolated:** An "isolated" nucleic acid is one that has been substantially separated or purified away from other nucleic acid sequences in the cell of the organism in which the nucleic acid naturally occurs, i.e., other chromosomal and extrachromosomal DNA and RNA, by conventional nucleic acid-purification methods. The term also embraces recombinant nucleic acids and chemically synthesized nucleic acids.

**Plant:** The term "plant" encompasses any higher plant and progeny thereof, including monocots (*e.g*., corn, rice, wheat, barley, rapeseed, soy, sunflower, *etc.*), dicots (*e.g*., potato, tomato, *etc*.), and includes parts of plants, including seeds, fruit, tubers, *etc*.

The invention will be better understood by reference to the examples herein. The scope of the invention is not to be considered limited thereto.

### Description And General Methods Of The Disclosure

The present invention utilizes standard laboratory practices for the cloning, manipulation, and sequencing of nucleic acids, the purification and analysis of proteins, and other molecular biological and biochemical techniques, unless otherwise stipulated. Such techniques are explained in detail in standard laboratory manuals such as Sambrook et al., *Molecular Cloning: A Laboratory Manual,* 2^{nd} ed., Cold Spring Harbor Laboratory Press, NY, 1989; and Ausubel et al., *Current Protocols in Molecular Biology,* Green and Wiley-Interscience, **NY,** 1987.

The inventors have identified, cloned, and expressed a novel fatty acid Δ⁸ - desaturase from the protist *Euglena gracilis* and a novel fatty acid Δ⁵-desaturase from *Caenorhabditis* elegans that may be used together to produce polyunsaturated fatty acids.

The invention provides novel purified proteins (FIG. 7A). The invention also provides proteins differing from the protein of FIG. 7A by one or more conservative amino acid substitutions, as Well as proteins that show "substantial similarity" with the protein of FIG. 7A. Substantial similarity is defined in the "Definition" section. Proteins of the invention include proteins that show at least 50% amino acid similarity with the protein shown in FIG. 7A. The term "50% amino acid similarity" is objectively and consistently defined by use of blastp sequence analysis software set at default parameters. Proteins of the invention also include proteins showing at least 60%, at least 70%, at least 80%, at least 90%, and at least 95% similarity (to the sequence of FIG. 7A) using blastp with default parameters.

The invention provides isolated novel nucleic acids that encode the above-mentioned proteins, recombinant nucleic acids that include such nucleic acids and cells containing such recombinant nucleic acids. Nucleic adds of the invention thus include nucleic acids that encode: (1) amino acid sequences as shown in FIG. 7A; (2) amino add sequences that differ from the Sequences shown in FIG. 7A by one or more conservative amino acid substitutions; and (3) amino acid sequences that show at least 60% similarity (as measured by blastp at default parameters) with the sequence of FIG. 7A.

Nucleic acids of the invention also include nucleic acids that show at least the term 60% similarity" with the nucleic acids shown in FIG. 7B. The term 60% similarity" is objectively defined by the use of blastn software set at default perimeters. Nucleic acids of the invention also include nucleic acids showing at least 70%, at least 80%, at least 90%, and at least 95% similarity (to the sequence of FIG.7B) using blastn with default perimeters.

The novel Δ⁸-desaturase enzymes can be used individually, or in conjunction with one another, for instance in a metabolic pathway, to produce polyunsaturated fatty acids, such as 20:3 and 20:4 fatty acids. FIG. 1B shows an example of such a metabolic pathway. Such a pathway may be engineered into any cell by use of appropriate expression systems. A simple way to provide such elements is by the use of commercially available expression systems, discussed in detail below.

The scope of the invention covers not only entire nucleic acids encoding the novel Δ⁸ desaturase enzymes (and substantially similar derivatives of such enzymes) but also covers "portions" of such nucleic acids (as defined in the "Definitions" Section, herein). Such claimed portions are identified by their possession of a particular degree of similarity with similar sized portions of the nucleotides of FIG. 7B and may have a length of about 15, 20, 30, 40, or 50 contiguous nucleotides. Similarity is objectively measured by sequence comparison software, such as the "blastn" and "blastp" software available from the National Center for Biotechnology Information (NBCI, Bethesda, MD) and on the Internet at htp://www.ncbi.nlm nih.gov/BLAST/. Similarity between portions of nucleic acids claimed and similar sized portions of the nucleic acid sequences of FIG. 7B may be at least 50%, 60%, 70%, 80%, 90%, 95%, or even 98%. Such portions of nucleic acids may be used, for instance, as primers and probes for research and diagnostic purposes. Portions of nucleic acids may be selected from any area of the sequences shown in FIG. 7B, for instance the first; second, third, etc., group of 100 nucleic acids as numbered in the figures.

Recombinant nucleic acid, as mentioned above, may, for instance, contain all or portion of a disclosed nucleic acid operably linked to another nucleic acid element such as a promoter, for instance, as part of a clone designed to express a protein. Cloning and expression systems are commercially available for such purposes.

Various yeast strains and yeast-derived vectors are commonly used for expressing and purifying proteins, for example, *Pichia pastoris* expression systems are available from Invitrogen (Carlsbad, CA). Such systems include suitable *Pichia pastoris* strains, vectors, reagents, sequencing primers, and media. A similar system for expression of proteins in *Saccharomyces cerevisiae* is also available from Invitrogen, which includes vectors, reagents and media. For example, a nucleotide sequence (e.g., a gene coding for the Δ⁵ - or Δ⁸-desaturase enzyme of the invention) may be cloned into the yeast expression vector pYES2 and expressed under the control of an inducible promoter, such as a galactose-inducible promoter (GAL1).

Non-yeast eukaryotic vectors may also be used for expression of the desaturases of the invention. Examples of such systems are the well known Baculovirus system, the Ecdysone-inducible mammalian expression system that uses regulatory elements from *Drosophila melanogaster* to allow control of gene expression, and the Sindbis viral expression system that allows high level expression in a variety of mammalian cell lines. These expression systems are also available from Invitrogen.

Standard prokaryotic cloning vectors may also be used, for example pBR322, *pUC18* or *pUC19* as described in Sambrook et al, 1989. Nucleic acids encoding the desaturases of the invention may be cloned into such vectors that may then be transformed into bacteria such as *Escherischia coli (E. coli)* which may then be cultured so as to express the protein of interest. Other prokaryotic expression systems include, for instance, the arabinose-induced pBAD expression system that allows tightly controlled regulation of expression, the IPTG-induced pRSET system that facilitates rapid purification of recombinant proteins and the IPTG-induced pSE402 system that has been constructed for optimal translation of eukaryotic genes. These three systems are available commercially from Invitrogen and, when used according to the manufacturer's instructions, allow routine expression and purification of proteins.

Alternatively, and of particular importance to this invention, a plant expression system could be used. Plant expression systems are commercially available. A gene of interest of the invention may be cloned into a vector and the construct used to transform a plant cell. Any well known vector suitable for stable transformation of plant cells and/or for the establishment of transgenic plants may be used, including those described in, e.g., Pouwels et al., *Cloning Vectors: A Laboratory Manual,* 1985, supp. 1987; Weissbach and Weissbach, *Methods for Plant Molecular Biology,* Academic Press, 1989; and Gelvin et al., *Plant Molecular Biology Manual,* Kluwer Academic Publishers, 1990. Such plant expression vectors can include expression control sequences (e.g., inducible or constitutive, environmentally or developmentally regulated, or cell- or tissue-specific expression-control sequences).

Examples of constitutive plant promoters useful for expressing desaturase enzymes in plants include, but are not limited to, the cauliflower mosaic virus (CaMV) 35S promoter (see, *e.g.,* Odel et al., *Nature* **313:**810, 1985; Dekeyser et al., *Plant Cell* **2:**591, 1990; and Terada and Shimamoto, *Mol. Gen. Genet.* **220**:389, 1990); the nopaline synthase promoter (An et al., *Plant Physiol.* **88**:547, 1988) and the octopine synthase promoter (Fromm et al., *Plant Cell* **1:**977, 1989).

A variety of plant-gene promoters that are regulated in response to environmental, hormonal, chemical, and/or developmental signals, also can be used for protein expression in plant cells, including promoters regulated by (1) heat (Callis et al., *Plant Physiol.* **88:**965, 1988), (2) light (e.g., pea rbcS-3A promoter, Kuhlemeier et al., *Plant Cell* **1**:471, 1989; maize rbcS promoter, Schaffner and Sheen, *Plant Cell* **3**:997, 1991; or chlorophyll a/b-binding protein promoter, Simpson et al., *EMBO J.* **4**:2723, 1985), (3) hormones, such as abscisic acid (Marcotte et al., *Plant Cell* **1**:969, 1989), (4) wounding (*e.g., wunl,* Siebertz et al., *Plant Cell* 1:961,1989); or (5) chemicals such as methyl jasmonate, salicylic acid, or a safener. It may also be advantageous to employ (6) organ-specific promoters (*e*.*g*., Roshal et al., *EMBO J*. **6**:1155, 1987; Schemthaner et al., *EMBO J.* **7:**1249, 1988; Bustos et al., *Plant Cell* 1:839, 1989; Zheng et al., *Plant J*. **4**:357-366, 1993). Tissue-specific expression may be facilitated by use of certain types of promoters, for example, the napin promoter is a seed-storage protein promoter from *Brassica* and specific to developing seeds. The β-conglycinin promoters drive the expression of recombinant nucleic acids thus allowing, the Δ⁵ or Δ⁸ proteins of the invention to be expressed only in specific tissues, for example, seed tissues.

Plant expression vectors can include regulatory sequences from the 3'-untranslated region of plant genes (Thornburg et al., *Proc*. *Natl. Acad*. *Sci*. USA **84**:744, 1987; An et al., *Plant Cell* **1**:115, 1989), *e.g*., a 3' terminator region to increase mRNA stability of the mRNA, such as the PI-II terminator region of potato or the octopine or nopaline syntase 3' terminator regions.

Useful dominant selectable marker genes for expression in plant cells include, but are not limited to: genes encoding antibiotic-resistance genes (*e.g.*, resistance to hygromycin, kanamycin, bleomycin, G418, streptomycin, or spectinomycin); and herbicide-resistance genes (e.g., phosphinothricin acetyltransferase). Useful screenable markers include, but are not limited to, β-glucuronidase and green fluorescent protein.

The invention also provides cells or plants or organisms transformed with recombinant nucleic acid constructs that include all or a portion of the newly discovered polynucleotides that encode the novel Δ⁸ desaturase enzymes. An example of such a transformed plant or organism would be a potato, tomato, rapeseed, sunflower, soy, wheat, or corn plant. Multi-celled fungi, such as edible mushrooms, may also be transformed. Transformed oil-seed plants are of particular interest as 20-carbon polyunsaturated fatty acids would accumulate within the seed-oil.

Nucleic acid constructs that express a nucleic acid according to the invention can be introduced into a variety of host cells or organisms in order to alter fatty acid biosynthesis. Higher plant cells, eukaryotic, and prokaryotic host cells all may be so transformed using an appropriate expression system as described above.

After a cDNA (or gene) encoding a desaturase has been isolated, standard techniques may be used to express the cDNA in transgenic plants in order to modify the particular plant characteristic. The basic approach is to clone the cDNA into a transformation vector, such that the cDNA is operably linked to control sequences (e.g., a promoter) directing expression of the cDNA in plant cells. The transformation vector is then introduced into plant cells by any of various techniques, for example by *Agrobacterium-*mediated transformation of plants or plant tissues, or by electroporation of protoplasts, and progeny plants containing the introduced cDNA are selected. All or part of the transformation vector stably integrates into the genome of the plant cell. That part of the transformation vector that integrates into the plant cell and that contains the introduced cDNA and associated sequences for controlling expression (the introduced "transgene") may be referred to as the "recombinant expression cassette."

Selection of progeny plants containing the introduced transgene may be made based upon the detection of an altered phenotype. Such a phenotype may result directly from the cDNA cloned into the transformation vector or may be manifested as enhanced resistance to a chemical agent (such as an antibiotic) as a result of the inclusion of a dominant selectable marker gene incorporated into the transformation vector.

Successful examples of the modification of plant characteristics by transformation with cloned cDNA sequences are replete in the technical and scientific literature. Selected examples, which serve to illustrate the knowledge in this field of technology include:
U.S. Patent No. 5,571,706 ("Plant Virus Resistance Gene and Methods")
U.S. Patent No. 5,677,175 ("Plant Pathogen Induced Proteins")
U.S. Patent No. 5,510,471 ("Chimeric Gene for the Transformation of Plants")
U.S. Patent No. 5,750,386 ("Pathogen-Resistant Transgenic Plants")
U.S. Patent No. 5,597,945 ("Plants Genetically Enhanced for Disease Resistance")
U.S. Patent No. 5,589,615 ("Process for the Production of Transgenic Plants with Increased Nutritional Value Via the Expression of Modified 2S Storage Albumins")
U.S. Patent No. 5,750,871 ("Transformation and Foreign Gene Expression in Brassica Species")
U.S. Patent No. 5,268,526 ("Overexpression of Phytochrome in Transgenic Plants")
U.S. Patent No. 5,262,316 ("Genetically Transformed Pepper Plants and Methods for their Production")
U.S. Patent No. 5,569,831 ("Transgenic Tomato Plants with Altered Polygalacturonase Isoforms")

These examples include descriptions of transformation vector selection, transformation techniques, and the construction of constructs designed to over-express the introduced cDNA. In light of the foregoing and the provision herein of the desaturase amino acid sequences and nucleic acid sequences, it is thus apparent that one of skill in the art will be able to introduce the cDNAs, or homologous or derivative forms of these molecules, into plants in order to produce plants having enhanced desaturase activity. Furthermore, the expression of one or more desaturases in plants may give rise to plants having increased production of poly-unsaturated fatty acids.

The invention also pertains to antibodies to the desaturase enzymes, and fragments thereof, these antibodies may be useful for purifying and detecting the desaturases. The provision of the desaturase sequences allows for the production of specific antibody-based binding agents to these enzymes.

Monoclonal or polyclonal antibodies may be produced to the desaturases, portions of the desaturases, or variants thereof. Optimally, antibodies raised against epitopes on these antigens will specifically detect the enzyme. That is, antibodies raised against the desaturases would recognize and bind the desaturases, and would not substantially recognize or bind to other proteins. The determination that an antibody specifically binds to an antigen is made by any one of a number of standard immunoassay methods; for instance, Western blotting , Sambrook et al. (ed.), *Molecular Cloning: A Laboratory Manual,* 2nd ed. Vols. 1-3, Cold Spring Harbor Laboratory Press, Cold Spring Harbor, NY, 1989.

To determine that a given antibody preparation (such as a preparation produced in a mouse against the Δ⁵ -desaturase) specifically detects the desaturase by Western blotting, total cellular protein is extracted from cells and electrophoresed on a SDS-polyacrylamide gel. The proteins are then transferred to a membrane (for example, nitrocellulose) by Western blotting, and the antibody preparation is incubated with the membrane. After washing the membrane to remove non-specifically bound antibodies, the presence of specifically bound antibodies is detected by the use of an anti-mouse antibody conjugated to an enzyme such as alkaline phosphatase; application of 5-bromo-4-chloro-3-indolyl phosphate/nitro blue tetrazolium results in the production of a densely blue-colored compound by immuno-localized alkaline phosphatase.

Antibodies that specifically detect a desaturase will, by this technique, be shown to bind substantially only the desaturase band (having a position on the gel determined by the molecular weight of the desaturase). Non-specific binding of the antibody to other proteins may occur and may be detectable as a weaker signal on the Western blot (which can be quantified by automated radiography). The non-specific nature of this binding will be recognized by one skilled in the art by the weak signal obtained on the Western blot relative to the strong primary signal arising from the specific anti-desaturase binding. Antibodies that specifically bind to desaturases belong to a class of molecules that are referred to herein as "specific binding agents." Specific binding agents that are capable of specifically binding to the desaturase of the present invention may include polyclonal antibodies, monoclonal antibodies, and fragments of monoclonal antibodies such as Fab, F(ab')2, and Fv fragments, as well as any other agent capable of specifically binding to one or more epitopes on the proteins.

Substantially pure desaturase suitable for use as an immunogen can be isolated from transfected cells, transformed cells, or from wild-type cells. Concentration of protein in the final preparation is adjusted, for example, by concentration on an Amicon filter device, to the level of a few micrograms per milliliter. Alternatively, peptide fragments of a desaturase may be utilized as immunogens. Such fragments may be chemically synthesized using standard methods, or may be obtained by cleavage of the whole desaturase enzyme followed by purification of the desired peptide fragments. Peptides as short as three or four amino acids in length are immunogenic when presented to an immune system in the context of a Major Histocompatibility complex (MHC) molecule, such as MHC class I or MHC class II. Accordingly, peptides comprising at least 3 and preferably at least 4, 5, 6, or more consecutive amino acids of the disclosed desaturase amino acid sequences may be employed as immunogens for producing antibodies.

Because naturally occurring epitopes on proteins frequently comprise amino acid residues that are not adjacently arranged in the peptide when the peptide sequence is viewed as a linear molecule, it may be advantageous to utilize longer peptide fragments from the desaturase amino acid sequences for producing antibodies. Thus, for example, peptides that comprise at least 10, 15, 20, 25, or 30 consecutive amino acid residues of the amino acid sequence may be employed. Monoclonal or polyclonal antibodies to the intact desaturase, or peptide fragments thereof may be prepared as described below.

Monoclonal antibody to any of various epitopes of the desaturase enzymes that are identified and isolated as described herein can be prepared from murine hybridomas according to the classic method of Kohler & Milstein, *Nature* **256:**495, 1975, or a derivative method thereof. Briefly, a mouse is repetitively inoculated with a few micrograms of the selected protein over a period of a few weeks. The mouse is then sacrificed, and the antibody-producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall, *Enzymol.* **70**:419, 1980, or a derivative method thereof. Selected positive clones can be expanded and their monoclonal antibody product harvested for use. Detailed procedures for monoclonal antibody production are described in Harlow & Lane, *Antibodies,* A *Laboratory Manual,* Cold Spring Harbor Laboratory, New York, 1988.

Polyclonal antiserum containing antibodies to heterogenous epitopes of a single protein can be prepared by immunizing suitable animals with the expressed protein, which can be unmodified or modified, to enhance immunogenicity. Effective polyclonal antibody production is affected by many factors related both to the antigen and the host species. For example, small molecules tend to be less immunogenic than other molecules and may require the use of carriers and an adjuvant. Also, host animals vary in response to site of inoculations and dose, with both inadequate or excessive doses of antigen resulting in low-titer antisera. Small doses (ng level) of antigen administered at multiple intradermal sites appear to be most reliable. An effective immunization protocol for rabbits can be found in Vaitukaitis et al., *J. Clin. Endocrinol. Metab.* **33**:988-991, 1971.

Booster injections can be given at regular intervals, and antiserum harvested when the antibody titer thereof, as determined semi-quantitatively, for example, by double immunodiffusion in agar against known concentrations of the antigen, begins to fall. See, for example, Ouchterlony et al., *Handbook of Experimental Immunology,* Wier, D. (ed.), Chapter 19, Blackwell, 1973. A plateau concentration of antibody is usually in the range of 0.1 to 0.2 mg/mL of serum (about 12 µM). Affinity of the antisera for the antigen is determined by preparing competitive binding curves using conventional methods.

Antibodies may be raised against the desaturases of the present invention by subcutaneous injection of a DNA vector that expresses the enzymes in laboratory animals, such as mice. Delivery of the recombinant vector into the animals may be achieved using a hand-held form of the Biolistic system (Sanford et al., *Particulate Sci. Technol.* **5**:27-37, 1987, as described by Tang et al., *Nature (London)* **356**:153-154, 1992). Expression vectors suitable for this purpose may include those that express the cDNA of the enzyme under the transcriptional control of either the human β-actin promoter or the cytomegalovirus (CMV) promoter. Methods of administering naked DNA to animals in a manner resulting in expression of the DNA in the body of the animal are well known and are described, for example, in U.S. Patent Nos. 5,620,896 ("DNA Vaccines Against Rotavirus Infections"); 5,643,578 (Immunization by Inoculation of DNA Transcription Unit"); and 5,593,972 ("Genetic Immunization"), and references cited therein.

Antibody fragments may be used in place of whole antibodies and may be readily expressed in prokaryotic host cells. Methods of making and using immunologically effective portions of monoclonal antibodies, also referred to as "antibody fragments," are well known and include those described in Better & Horowitz, *Methods Enzymol.* **178**:476-496, 1989; Glockshuber et al. *Biochemistry* **29**:1362-1367, 1990; and U.S. Patent Nos. 5,648,237 ("Expression of Functional Antibody Fragments"); No. 4,946,778 ("Single Polypeptide Chain Binding Molecules"); and No. 5,455,030 ("Immunotherapy Using Single Chain Polypeptide Binding Molecules"), and references cited therein.

### Experimental Examples

### Example 1: Organism Strains and Culture

The strain *Euglena gracilis* Z was obtained from Columbia Scientific. The organism was cultured on Cramer and Meyers medium (Cramer, and Meyers, *Archiv fur Mikrobiologie* 17:384-402, 1952) with the addition of sucrose as a carbon source. Cultures were maintained at 25°C in absolute darkness.

C. *elegans* was obtained from Caenorhabditis Genetics Center, St. Paul, Minnesota, and grown under standard conditions (Sulston et al., *The Nematode Caenorhabditis elegans* (Wood, W. B., Eds.), pp. 587-606, Cold Spring Harbor Laboratory Press, Cold Spring Harbor, NY, 1988).

### Example 2: Database Searches for C. elegans Gene Homologs

The Sanger Center (http://www.sanger.ac.uk/projects/c_elegans/blast_server.shtml) *C. elegans* genomic database was searched using BLAST^{™} with sequences of plant desaturase enzymes, including the B. *officinalis* Δ⁶ -desaturase (GenBank accession number U7901 0). Two C. *elegans* polypeptides with the highest scores were a peptide on cosmid W08D2 (high score 163), and one on T13F2 (high score 121).

### Example 3: RNA Isolation, Reverse Transcription PCR, and RACE (Rapid Amplification of cDNA Ends)

For *the E. gracilis* Δ⁸ gene, total RNA was isolated from heterotrophic cultures of *E. gracilis* using a phenol-SDS protocol (Ausubel, *Current Protocols In Molecular Biology,* 1988). Messenger RNA was purified from total RNA using the PolyA-tract system (Promega Scientific, Madison, Wisconsin). Reverse transcription reactions were carried out using Superscript II (Life Technologies, Rockville, Maryland). First-strand synthesis in the initial reactions was primed using anchored polyT primers (Clontech, Palo Alto, California). Second-strand synthesis was conducted as described (Life Technologies), and polymerase chain reaction amplification of the core region of the gene was accomplished using the primers (GGCTGGCTGACNCAYGARTTYTGYCAY; SEQ. ID NO. 5) and (CATCGTTGGAAANARRTGRTGYTCDATYTG; SEQ. ID NO. 6), designed to be completely degenerate to sequences overlapping the first and third His-box regions of the Δ⁶-desaturase C. *elegans* gene.

The amplification protocol was developed using published guidelines for use of degenerate primers (Compton, *PCR Protocols: A Guide To Methods And Applications,* 1990). The amplification consisted of 5 preliminary cycles at very low annealing temperature (30 seconds at 94°C, 1-minute ramp to 37°C, 45 seconds at 37°C, 3-minute ramp to 72°C) followed by 30 cycles with higher temperature (30 seconds at 94°C, 1-minute ramp to 50°C, 45 seconds at 50°C, 3-minute ramp to 72°C. Preliminary amplifications to optimize thermal cycling parameters used *Pfu* DNA polymerase (Stratagene, La Jolla, California). Amplification was successful at 3 mM magnesium and each primer at 4 µM. Subsequently Taq polymerase was used for amplification under identical conditions.

Polymerase chain reaction products from 350 to 750 bp were isolated from agarose gels with commercial reagents (Qiagen, Valencia, California) and sequenced directly using the degenerate primers and dye-termination sequencing technology (Applied Biosystems, Foster City, California). A group of identical amplification products contained an open reading frame that was homologous to known desaturases when analyzed by BLAST^{™} search (Altschul et al., *Nucleic Acids Res.* **25**:3389-3402, 1997). The 5' and 3' sequences of the complete mRNA were obtained with the Marathon RACE system (Clontech), using pairs of nested primers designed to amplify from within the core sequence. To clone the complete 5' end of the gene, it was necessary to repeat the reverse transcription with a primer specific to the sequence of the open reading frame and repeat the 5' RACE amplification.

For the C. *elegans* Δ⁵ gene, RNA isolation and reverse transcription-PCR were performed as follows. RT-PCR was used to amplify the coding sequences of the two putative desaturase genes. Total RNA from mixed stage C. *elegans* was used for the RT-PCR template. The nematodes were grown on agar plates as described and RNA was isolated using the phenol/SDS method (Sluder et al., Dev. *Biol.* **184**:303-319, 1997). RT-PCR was performed using the Superscript^{™} One-Step RT-PCR system (Gibco-BRULife Technologies). Approximately 1 µg of total RNA was added to a reaction mixture consisting 0.2 mM of each dNTP, 1.2 mM MgSO₄, Superscript II^{™} RT/Taq polymerase mix, and 200 µM of appropriate downstream and upstream primers. The reactions were incubated at 50°C for 30 min., then subject to 35 cycles of PCR amplification. For the T13F2.1 gene (*fat-*4) a 5' primer corresponding to bases 34339-34361 of cosmid T13F2 was used. *Sma*I, *Hind*III, and Xhol restriction sites were added to these sequences to facilitate cloning. The resulting primer [CCCGGGAAGCTTCTCGAGGAATTTTCAATCCTCCTTGGGTC; SEQ. ID NO: 7] anneals to the cosmid T13F2 19-42 base pairs upstream of the putative start codon ATG of the *fat-4* gene. To amplify the 3' end of the *fat-4* gene, a primer was used corresponding to the inverse complement of bases 37075-37095 of cosmid T13F2, with the addition of *Sma*I and *Bam*H1 sites to facilitate cloning the polynucleotide of interest: CCCGGGTGGATCCGGAACATATCACACGAAACAG; SEQ. ID NO. 8]. This primer begins 93 base pairs after the putative stop codon TAG and ends 20 base pairs upstream of the predicted polyadenylation signal (AAUAAA; SEQ ID NO: 9) of the *fat-4* gene.

For the determination of trans-splicing of specific leader sequences, downstream primers corresponding to the complement of bases 35009-35028 of the T13F2 (TCTGGGATCTCTGGTTCTTG; SEQ. ID NO: 10) were used for the T13F2.1 gene. The upstream PCR primers were either SL1-20 or SL2-20 (Spieth et al., *Cell* **73:**521-532, 1993). The *C. elegans* homologue of the ribosomal-protein L37 was used as an SL1-specific control, and K06H7.3 was used as an SL2-specific control (Zorio et al., *Nature* **372**:270-272, 1994. SL1-20, SL2-20, and control primers were kindly provided by Diego A. R. Zorio. RT-PCR products visualized by gel electrophoresis were confirmed by blotting the gel and probing with gene-specific oligonucleotides corresponding to the appropriate gene as previously described (Spieth et al., *Cell* **73**:21-532, 1993).

### Example 4: PCR Amplification of the Genes Encoding Δ⁵ and Δ⁸ Desaturases

DNA and protein sequences were analyzed using the Wisconsin-GCG package of programs (Devereux et al., *Nucleic Acids Res.* **12:**387-95, 1984).

To clone the *E*. *gracilis* (Δ⁸) open reading frame as a single DNA fragment, a set of primers was used to prime a reverse transcription specifically for the open reading frame. The primer for the 5' end of the gene began 3 nucleotides before the start codon and included the first 26 nucleotides of the open reading frame. The 3' primer was complementary to the sequence between 22 and 52 nucleotides downstream from the predicted termination codon. This PCR amplification was conducted with Pfu polymerase to minimize the chance of an amplification error. The PCR reactions produced a single band of the predicted size when analyzed by agarose gel electrophoresis. This band was cloned into the vector pCR-Script Cam^{™} (Stratagene), and a single clone designated pJW541 was chosen for analysis.

To express the *C. elegans* Δ⁵-desaturase, the *fat-4* cDNA amplification product (see Example 3) was digested with *Hind*III and *Bam*H1 and ligated to the yeast expression vector pYES2 (Invitrogen) cut with *Hind*III and *Bam*H1. The resulting plasmid was named pYFAT4.

### Example 5: Expression of Δ⁵ and Δ⁸-desaturases

For *E*. *gracilis,* the cloned Δ⁸ gene was transferred to the yeast expression vector pYES2 (Invitrogen, Carlsbad, California) by standard cloning techniques (Ausubel, *Current Protocols In Molecular Biology,* 1988) using enzymes obtained from New England Biolabs, Beverly, Massachusetts. The resulting yeast expression construct containing the open reading frame under the control of a galactose-inducible promoter was designated pYES2-541.

*Saccharomyces cerevisiae* strain INVSc1 (Invitrogen) was transformed with pYES2-541 and cultured using standard methods (Ausubel, *Current Protocols In Molecular Biology,* 1988). Liquid medium containing 2% galactose was supplemented with fatty acid soaps (NuCheck Prep, Elysian MN) at a final concentration of 0.2 mM. Tergitol (1%, NP40) was added to the yeast cultures to enhance fatty acid uptake (Stukey et al., *J. Biol. Chem.* 264:16537-16544, 1989), except for cultures containing 20:1, where 5% DMSO was substituted. Yeast were incubated overnight at 28°C , harvested by centrifugation, washed once with 1% Tergitol, once with 0.5% Tergitol, and finally once with distilled water.

For the *C*. *elegans* Δ⁵ gene, the constructs were transformed into *Saccharomyces cerevisiae* strain INVSc1 using the S.c. EasyComp transformation kit (Invitrogen). For experiments with the FAT-4 peptide, transformed yeast were grown overnight in uracil-deficient media containing 2% galactose, 0.2 mM fatty acid, and 1% NP-40. Under these conditions the percentage of these supplemented fatty acids which were incorporated into yeast lipids ranged from 14-28% of the total yeast fatty acids. For experiments in which 20:1Δ¹¹ was used as a substrate, the 1% NP-40 was replaced by 5% DMSO to achieve better incorporation of this fatty acid.

### Example 6: Analysis of Fatty Acids Using Gas Chromatography and GC-Mass Spectrometry

Extraction of lipids and preparation of fatty acid methyl esters was carried out by standard methods (Miquel and Browse, *J. Biol. Chem.* **267**:1502-1509, 1992). Gas chromatography of the methyl esters was conducted by established methods (Spychalla et al., *Proc. Natl. Acad. Sci. USA* **94**:1142-1147, 1997). Fatty acid 4,4-Dimethyloxazoline (DMOX) derivatives of yeast lipid extracts were prepared by standard methods (Fay and Richli, *J. Chromatogr.* **541**:89-98, 1991). GC-mass spectrometry was conducted on a Hewlett-Packard 6890 series GC-MS fitted with a 30m x 0.25 µm HP5MS column, operating at an ionization voltage of 70 eV with a scan range of 50-550 Da. Fatty acids and their derivatives were identified where possible by comparison with authentic standards (NuCheck Prep).

### Example 7: Identification and Amplification of the Euglena Δ⁸ -desaturase Gene

Messenger RNA isolated from heterotrophic cultures was used as template for reverse transcription followed by PCR amplification using degenerate primers that spanned the first and third conserved histidine-rich regions of microsomal desaturase proteins. The *C. elegans* Δ⁶-desaturase gene, FAT-3, was used as the principal basis for primer design. To compensate for the high degeneracy necessary in the primer pair, amplification reactions began with five cycles of low-temperature annealing and a long temperature ramp between the annealing and polymerization steps. Preliminary amplifications to optimize thermal cycling parameters used the proofreading Pfu DNA polymerase. After successful Pfu amplification reactions using high primer and magnesium concentrations, *Taq* polymerase was used to generate a number of bands detectable on agarose gels.

Several of these bands, of approximately 650 bp, had identical sequence. This DNA sequence contained an open reading frame in which the predicted amino acid sequence was homologous to other membrane desaturases, and included a characteristic central His-box. Primers designed to be specific to the amplified sequence were used to amplify the termini of the cDNA using 3' and 5' RACE techniques. The full-length cDNA for this gene was 1745 bp in length. It included an open reading frame of 1272 bp and a 472-bp 3' untranslated region. Most *Euglena* messenger RNAs are processed through the addition of a short 5' RNA leader sequence, the trans-spliced leader (Tessier et al., *Embo. J*. **10**:2621-2625, 1991). This RNA processing step left a conserved sequence (TTTTTTTCG; SEQ. ID NO. 11) at the beginning of each message (Cui et al., *J*. *Biochem.* (Tokyo) **115**:98-107, 1994). The presence of this leader in the cDNA sequence confirmed that the message was full-length at the 5' end. RT-PCR with primers flanking the open reading frame on the 5' and 3' ends resulted in a single band that was cloned into the vector pCR-Script Cam^{™} (Stratagene), and designated pJW541. The gene corresponding to this ORF was designated EFD1 *(Euglena* fatty acid desaturase 1).

### Example 8: Similarity Between Euglena Δ⁸ -desaturase and Other Proteins

The translated open reading frame indicated a protein of 422 amino acids with a predicted molecular mass of 48.8 kDa. (FIG. 3). A BLAST^{™} search of sequence databases revealed that the predicted protein sequence exhibited regions of homology with the known group of membrane fatty acid desaturases, especially in the highly conserved histidine-rich regions (Shanklin et al., *Biochemistry* **33**:12787-12794, 1994).

Each of the His-box motifs is present in the EFD1 protein. The first (HXXXH) starts at amino acid 146 and the second (HXXHH; SEQ ID NO: 12) at amino acid 183 (FIG. 3). EFD1 contains a variant third His-box, QXXHH (SEQ ID NO: 13), starting at amino acid 361, similar to the cloned Δ⁵- and Δ⁶ -desaturases. EFD1 exhibits conservation of protein sequence in the regions surrounding the highly conserved regions, especially with FAT-3 and FAT-4, the Δ⁶- and Δ⁵-desaturases of *C. elegans* (FIG. 3). Outside the highly conserved regions, the amino acid sequence shows considerably less similarity to other desaturases. Overall, the amino acid identity with FAT-3 and FAT-4 is 33%, compared to 28% identity with the borage Δ⁶-desaturase.

EFD1 also contains a cytochrome b₅-like motif at its N-terminus. The protein encodes seven of the eight most highly conserved amino acids characteristic of cytochrome b₅ (FIG. 3), which are responsible for heme binding. Similar motifs are found at the N-terminal regions of FAT-3 and FAT-4 (FIG. 3), and the borage Δ⁶ protein, as well as the carboxyl terminus of a yeast Δ⁹ protein.

The structure of the *Euglena* protein also exhibits similarities with known desaturases. Membrane desaturases are type II multiple membrane spanning proteins, and hydropathy analysis of the cloned *Euglena* gene indicates that the predicted protein has at least three significant hydrophobic regions long enough to span the membrane bilayer twice. As is true for most desaturase enzymes, there are 31 amino acid residues between the first two His-boxes. The distance between the between the second and third His-box is 173 residues, within the range previously observed (Shanklin and Cahoon, *Annu. Rev. Plant Physiol. Plant Mol. Biol.* **48:**611-641, 1998).

### Example 9: Activity of the Euglena Δ⁸ -desaturase Protein

To confirm the activity of the enzyme, the *EFD1* cDNA was transferred from pJW541 to yeast expression vector pYES2 under the control of a galactose-inducible promoter. The resulting construct, pYES2-541, was introduced into *S. cerevisiae.* Yeast membranes do not contain 20-carbon fatty acids but incorporate them from the culture medium. Accordingly, yeast cultures were supplemented with various fatty acid soaps, using a yeast strain containing the empty vector as control, and analyzed the fatty acids of the cultures by methyl-ester derivatization and gas chromatography.

The patterns of desaturation activity in these experiments indicated that pYES2-541 expresses a Δ⁸-desaturase enzyme that does not have Δ⁵ or Δ⁶ activity. The ability of the experimental yeast strain to produce Δ⁸ desaturation was shown when the culture medium was supplemented with 20:2 (FIG. 4). A desaturation peak whose retention time is identical to authentic 20:3 was produced. The vector-only control culture did not desaturate 20:2 (FIG. 4). The yeast strain expressing the *Euglena* gene also desaturated 20:3 and 20:1 (FIG. 4), again without desaturation activity in the control cultures. The cloned *Euglena* protein was most active with 20:3 and 20:2 as substrates, desaturating 70% and 73% of the total incorporated 20-carbon fatty acid. EFD1 was least active with 20:1, converting 32% of that substrate to a desaturation product (FIG. 4).

When the culture medium was supplemented with a substrate for Δ⁵-desaturation, 20:4, the fatty acid was incorporated into the yeast, but no 20:5 desaturation product was produced. Similarly, when the medium was supplemented with 18:2 and 18:3, no desaturation occurred, demonstrating that the cloned gene did not express a Δ⁶-desaturase.

To confirm that desaturation had occurred at the Δ⁸ position, 4,4-dimethyloxazoline (DMOX) derivatives of yeast fatty acids were analyzed by GC-MS. DMOX derivatives have mass spectra that are more easily interpreted than spectra of methyl esters, and permit unambiguous determination of double-bond locations in polyunsaturated fatty acids (Christie, *Lipids* **33**:343-353, 1998). For the experiment that desaturated the fatty acid 20:2, the retention time of the product on the GC-MS instrument was 16.8 min., identical to DMOX-derivatized authentic 20:3. The mass spectrum of this desaturation product and its molecular ion (m/z 359) indicated that it was the 20:3 compound. Two spectral frequency peaks at m/z 182 and 194, separated by only 12 a.m.u., showed that the introduced double bond was at the Δ⁸ position (FIG. 5). (The substrate 20:2, which is saturated at the 8-position, has peaks at 182 and 196, separated by 14 a.m.u.) The spectrum of the product, with its desaturation peaks, was identical to that of authentic 20:3 (Luthria and Sprecher, *Lipids* **28**:561-564, 1993). The other substrates, 20:1 and 20:3, were also desaturated at the Δ⁸-position by EFD1. The peaks at *mlz* 182 and *mlz* 194 appeared in the spectrum of each, and the molecular ion was reduced from that of the substrate by two in each case (FIG. 5).

### Example 10: Identification and Cloning of Two Fatty Acid C. elegans Desaturase Genes

Two high-scoring open reading frames were identified during a search of the *C. elegans* genomic DNA database with the borage Δ⁶-desaturase protein sequence. Both proteins predicted from these open reading frames, W08D2.4 and T13F2.1, contained an N-terminal sequence resembling cytochrome b₅, including the characteristic (HPGG) heme binding domain, and an H → Q substitution in the third histidine box. The W08D2.4 gene was denoted *fat-3* and the T13F2.1 gene was denoted *fat-4*, since they both appear to encode fatty acid desaturases. Interestingly, the *fat-3* and *fat-4* genes are located next to each other on overlapping cosmids in the same 5' to 3' orientation, with only 858 nucleotide base pairs separating the putative polyadenylation signal of the *fat-4* gene and the ATG start codon of the *fat-3* gene (FIG. 8). This gene organization is reminiscent of operons, in which two or more genes are transcribed under the control of a single promoter and regulatory region.

In C. *elegans* the polycistronic pre-mRNA is converted to monocistronic mRNA by cleavage and polyadenylation at the 3' end of the upstream gene and transplicing to the SL2 sequence at the 5' end of the downstream gene, with the two mRNAs being subsequently independently translated. However, out of more than 30 such operons that have been analyzed, the distances between the 3' end of the upstream gene and the 5' end of the downstream gene are generally about 100 base pairs, with a few separated by 300-400 base pairs (Blumenthal et al, *C. elegans* II, pp. 117-145, Cold Spring Harbor Laboratory Press, Cold Spring, NY, 1997).

The *C. elegans fat-3* and *fat-4* genes were tested to determine whether they were trans-spliced to either SL1 or SL2 in order to determine if they might be co-transcribed in a single operon. It was found that the *fat-4* gene was transpliced to SL1, but that the *fat-3* gene was not transpliced to either spliced leader sequence. Therefore, it was concluded that each gene contains its own 5' promoter and regulatory region.

Both of these genes were cloned using RT-PCR. The *fat-4* gene sequence matched the T13F2 genomic sequence exactly. However the gene product encoded by the cDNA was seven amino acids shorter than predicted by Genefinder for T13F2.1 (GenBank accession number Z81122) because the DNA sequence encoding amino acid residues 198-204 was not present in the *fat-4* cDNA The resulting peptide length was 447 amino acids instead of the previously predicted 454 amino acids. The gene product encoded by the *fat-3* cDNA also matched the genomic sequence of W08D2.4 (GenBank accession number Z70271) perfectly. However, the gene product was also shorter than the predicted protein sequence. Codons for amino acid residues 38-67 of W08D2.4 were not present in the cDNA in both cases the gene-prediction software used in the genomic sequencing project appeared to have misidentified some intron DNA as coding sequences.

### Comparative Example 11: Sequence Comparisons for C. elegans Δ⁵

The *C*. *elegans* FAT-3 and FAT-4 proteins, the *Mortierella alpina* Δ⁵-desaturase, and the *B. officinalis* Δ⁶-desaturase appear to be proteins of similar structure in that they all contain an N-terminal cytochrome *b₅* domain, three histidine boxes, and distinct hydrophobic membrane-spanning domains predicted by the TMHMM program from the Center for Biological Sequence -Analysis, Technical University of Denmark (http://www.cbs.dtu.dk/services/TMHMM-1.0/). The predicted structure is consistent with the proposed desaturase structural model (Stukey et al., *J*. *Biol. Chem.* **265**:20144-20149, 1990). Despite these similarities, the overall sequence identity among the four proteins is quite low. For example, the FA-T-3 Δ⁶-desaturase and the borage Δ⁶-desaturase share only 28% identity on the amino acid level. The *fat-4* gene product shares 25% amino acid identity with the borage Δ⁶-desaturase and 19% amino acid identity with the *Mortierella alpina* Δ⁵-desaturase. Indeed the only portion of the FAT-4 protein that shows extended homology to the *M*. *alpina* Δ⁵-desaturase is a sequence of 36 residues incorporating the third His box which has 44% identity and 56% similarity. The most closely related pair of sequences are *fat-3* and *fat-4,* which are 46% identical on the amino acid level and 54% identical over the entire cDNA sequence.

FIG. 9 shows the sequence comparison of the borage Δ⁶-desaturase, *C. elegans* FAT-3, *C*. *elegans* FAT-4, and the *Mortierella alpina* Δ⁵-desaturase. The similar heme-binding domains (HPGG) and the three histidine box regions are underlined. The presence of these conserved motifs indicate that the *fat*-4 gene may encode a desaturase or a related fatty acid modifying enzyme. However it is not possible, from these sequence comparisons alone, to predict whether this gene encodes a Δ⁶ desaturase, a Δ⁵ -desaturase, or a more distantly related enzyme.

### Comparative Example 12: Fatty Acid Desaturase Activity and Substrate Specificity in Yeast for C. elegans Δ⁵

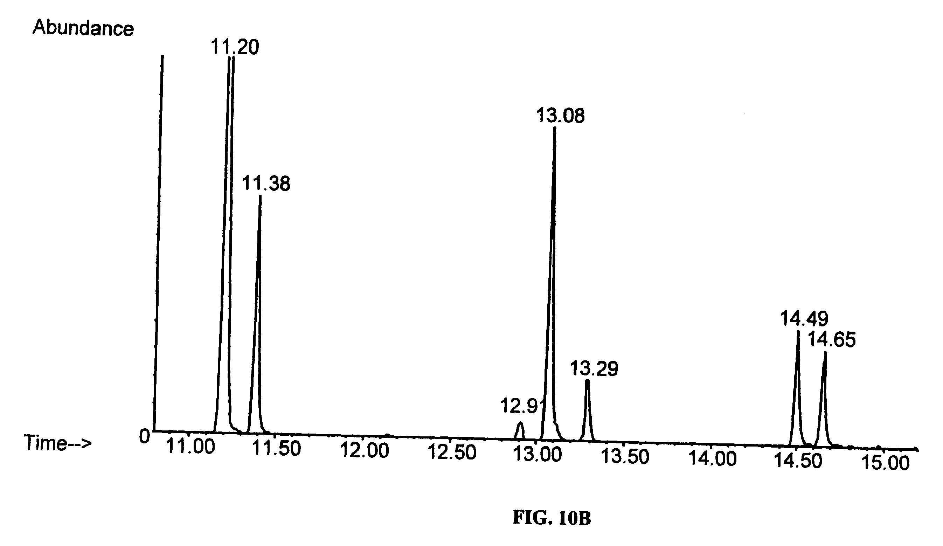

To determine the enzymatic activity of the FAT₋₄ desaturase-like protein, the protein was expressed in *Saccharomyces cerevisiae* supplemented with polyunsaturated fatty acid substrates that are not normally present in this yeast The FAT-4 protein was expressed in the yeast expression vector pYES2 from the GAL1 promoter by growing the cells in the presence of galactose and various fatty acids. After 16 hours of growth, the cells were analyzed for total fatty acid composition by gas chromatography (GC). Comparison of cells supplemented with di-homo-γ-linolenic acid (20:3Δ^{8,11,14}) carrying pYES2 containing the *fat-4* coding sequence and cells carrying the vector alone revealed the presence of a major new peak eluting at 14.49 minutes in the cells expressing FAT-4 (FIG. 10B). The novel peak had a retention time identical to that of the authentic arachidonic acid methyl ester (20:4Δ^{5,8,11,14} ), and was determined to be arachidonic acid (20:4Δ^{5,8,11,14}) because its mass spectrum was identical to that of authentic arachidonic acid methyl ester, including a mass ion peak at *m*/*z* 318.

The identity of this compound was further verified by converting the yeast fatty acid methyl esters into oxazoline derivatives in order to produce structure specific mass spectra which simplify the determination of double-bond positions in a hydrocarbon chain. The mass spectrum of the DMOX derivative of the novel 20:4 component was consistent with the published spectrum for arachadonic acid and contained a prominent peak at *m*/*z* 153, which is diagnostic of a double bond at the Δ⁵ position. Therefore, it was concluded that the *fat-4* gene encodes a Δ⁵-desaturase capable of synthesizing arachidonic acid from the substrate di-homo-γ-linolenic acid. In contrast, the FAT-4 protein showed no activity when linoleic acid (18:2Δ^{9,12}) or γ-linolenic acid (18:3Δ^{9,12,15}) were provided as substrates, indicating an absence of Δ⁶⁻desaturase activity.

Further analysis of the GC trace of the total fatty acids of the yeast cells expressing fat-4 revealed the presence of a second novel peak eluting at 12.91 minutes which was not present in the empty vector control cells. Analysis of the mass spectrum of this novel peak revealed a molecular ion species of 294, identical to that of a methyl ester of an 18-carbon fatty acid with two double bonds (18.2), buts its retention time and mass spectrum were not identical to the common isomer 18:2 Δ^{9,12}

In microsomal extracts of mammalian liver, Δ⁵ -desaturase activity has been reported to act on a number of 18 and 20-carbon precursors to produce uncommon fatty acids such as 18:20Δ^{5,11} ,20:3Δ^{5,11,14} and 20:4Δ^{5,11,14,17} (28, 29). Two species of slime molds have also been reported to produce small amounts of 18:2Δ^{5,9}, 18:2Δ^{5,11}, 20:3Δ^{5,11,14} and 20:4Δ^{5,11,14,17} (Rezanka, *Phytochemistry,* **33**:1441-1444, 1993). These fatty acids are unusual in that their double bonds do not follow the conventional methylene-interrupted pattern (one double bond every three carbons).

Therefore, it was suspected that the novel peak exhibited on the GC spectrum is a result of the *C. elegans* Δ⁵ -desaturase acting on 18:1Δ⁹ [or 18:1Δ¹¹, which in *S. cerevisiae* constitutes 15-20% of the total 18:1] compound, to produce the uncommon isomer 18:2Δ^{5,9} or 18:2Δ^{5,11}. These yeast fatty acid methyl esters were converted into oxazoline derivatives. It was found that the mass spectrum of the DMOX derivative of the novel 18:2 component contained the Δ⁵ -specific peak at *mlz* 153. However the larger ion peaks characteristic of double bonds at the Δ⁹ or Δ¹¹ position were not detected due to the small amount of this molecule present in the total yeast extracts.

To test if the *C. elegans* Δ⁵-desaturase was capable of desaturating other substrates to produce other uncommon, non-methylene-interrupted fatty acids, the yeast expressing the FAT-4-desaturase was supplemented with unconventional Δ⁵ -substrates such as 20:1Δ¹¹, 20:2Δ^{11,14}and 20:3Δ^{11,14,17}. No novel peaks were detected when the substrate 20:1Δ¹¹ was fed to yeast. However, when 20:2Δ^{11,14} and 20:30^{11,14,17} were provided as substrates, novel peaks were detected eluting at 14.62 minutes and 14.69 minutes, respectively (FIGS. 11A and 11 B, respectively). The mass-spectrum analysis of DMOX derivatives of these molecules yielded results consistent with published values for 20:3Δ^{5,11,14} and 20:4Δ^{5,11,14,17}, including a prominent ion peak of *m*/*z* 153 (which is diagnostic of double bonds at the Δ⁵ position). It was found, however, that these fatty acids were not produced to the same extent as arachidonic acid (20:4Δ^{5,8,11,14}) (FIG. 12). In these experiments, 55% of exogenously fed di-homo-γ-linolenic acid (20:3Δ^{8,11,14}) was converted to arachidonic acid, while only 5%, 27%, and 26% of the 18:1, 20:3Δ^{11,14}, and 20:2Δ^{11,14,17} substrates were converted (FIG. 12).

The *fat-3* gene was expressed in the yeast expression vector pMK195 containing the constitutive ADH promoter. The FAT-3 protein was able to desaturate linoleic acid (18:2Δ^{9,12}) into γ-linolenic acid (18:3Δ^{6,9,12}), in agreement with published results (Napier et al., *Biochem. J*. **330**:611-614, 1998). It was also found that FAT-3 was capable of desaturating α-linolenic acid (18:3Δ^{9,12,15}) to 18:4Δ^{6,9,12,15} , a common reaction in animals. The FAT-3 protein showed no activity on 20:1Δ¹¹ , 20:2Δ^{11,14}, 20:3Δ^{8,11,14}, or 20:3Δ^{11,14,17}. Therefore, the substrate specificities of the *C. elegans* Δ⁵ and Δ⁶-desaturases were determined to be specific and non-overlapping.

### Example 13: Discussion of the E. gracilis Δ⁸ -desaturase.

Desaturation at the Δ⁸-position has not been reported for any previously cloned gene (Tocher et al., *Prog. Lipid Res.* **37**:73-117, 1998).

The predicted EFD1 protein has 33% amino acid identity with both FAT-3 and FAT-4, (FIG. 9), while its identity with the cloned borage Δ⁶-desaturase is 28%. The highest sequence conservation is found in the His-box motifs which are critical for desaturase activity, most likely because they serve as the diiron-oxo component of the active site (Shanklin et al., *Biochemistry* **33**:12787-12794, 1994). Sequence conservation is also evident in the N-terminal cytochrome b₅-like domain, where most of the essential residues of cytochrome b₅ (Lederer, *Biochimie* **76**:674-692, 1994) that are retained in FAT-3 and FAT-4 are also present in the EFD1 protein (FIG. 3).

Expression of the *EFD1* gene in yeast was used to characterize its activity. Three different 20-carbon substrates with double bonds at the Δ¹¹-position were desaturated (FIG. 4), and analysis of the products indicated that, for each one, desaturation had taken place at the Δ⁸ position (FIG. 5). The cloned *Euglena* desaturase showed a clear preference for the substrates of metabolic significance with greater than two-fold preference for 20:2 and 20:3 over 20:1 (Ulsamer et al., *J. Cell Biol.* **43**:105-114, 1969). Even though EFD1 is quite similar to other microsomal desaturases, its activity was specific, as evidenced by its inactivity on substrates for Δ⁵ and Δ⁶ desaturation (FIG. 12).

The 20-carbon substrates for Δ⁸ desaturation are available in abundance in heterotrophically grown *E*. *gracilis* (FIG. 2). These same substrates also are available in mammals, since 20:2 and 20:3 are produced by elongation from 18:2 and 18:3, in competition with the typical Δ⁶ desaturation (FIG. 1). Labeling experiments with rat liver homogenates indicate that elongation of the 18-carbon fatty acids is five-fold more rapid than the competing desaturation (Pawlosky et al., *J*. *Lipid Res.* **33**:1711-1717, 1992).

Implicit in the current understanding of the Δ⁶ pathway of 20-carbon polyunsaturated fatty acid biosynthesis is a reliance on alternating desaturation and elongation to control flux through the pathway. While elongation often appears to be non-specific, most desaturations are specific both as to chain length of the substrate and to existing desaturation pattern of the fatty acid (Heinz, *Lipid Metabolism in Plants,* pp. 33-89, 1993). However, data from experiments in mammalian tissue (Bernert and Sprecher, *Biochim. Biophys. Acta.* **398**:354-363, 1975; Albert et al., *Lipids* **14**:498-500, 1979) and with yeast expressing the *C*. *elegans* Δ⁵ -desaturase gene (FIG. 12), indicate that Δ⁵ enzymes desaturate fatty acids having a double bond at the Δ¹¹-position but not at the Δ⁸-position, producing the non-methylene-interrupted 20:3 and 20:4 compounds at significant rates. For the Δ⁸ pathway, Δ⁸ desaturation occurs in competition with Δ⁵ activity on the substrates 20:2 and 20:3. In spite of this promiscuity of Δ⁵ enzymes, lipid profiles of mammalian tissue do not contain fatty acids with the Δ^{5,11} (Heinz, *Lipid Metabolism in Plants,* pp. 33-89, 1993; and Ulsamer et al., *J. Cell Biol.* **43:**105-114, 1969) desaturation pattern, nor are they seen in *Euglena.*

One explanation may be that Δ⁸-desaturation of the common substrates occurs very rapidly, while Δ⁵ -desaturation proceeds more slowly, so that little Δ^{5,11} product is formed. In support of this explanation, the *Euglena* Δ⁸ appears to be a very active desaturase when expressed in yeast (FIG. 4), compared to similarly expressed Δ⁵ -desaturase enzymes. The *Euglena* desaturase must be sufficiently active to account for all the long chain polyunsaturates of rapidly growing *Euglena* cultures (FIG. 2). In contrast, the observed rates of Δ⁸-desaturation in mammalian tissue are relatively slow. The highest apparent rate occurs in cancerous tissues without Δ⁶ activity, where Δ⁸ desaturation permits production of arachidonic acid at only 17% of the level of comparable normal cells with Δ⁶ activity (Grammatikos et al., *Br*. *J. Cancer* **70**:219-227, 1994).

Alternatively, the lack of Δ^{5,11} unsaturated fatty acids in membranes could be explained if the Δ⁸ desaturase accepts these fatty acids for desaturation. The convention that Δ⁸ -desaturation precedes Δ⁵ activity (FIG. 1) is based on observations of desaturation reactions proceeding sequentially along the fatty acid hydrocarbon chain. The reverse order of desaturation, with Δ⁵- saturation preceding Δ⁸-desaturation, has been claimed (Takagi, *J. Chem. Bull. Japan* **38**:2055-2057, 1965) and rejected (Schlenk et al., *Lipids* **5**:575-577, 1970) in mammalian liver, and proposed as a likely pathway based on experiments with deuterated substrates in glioma cells (Cook et al., *J*. *Lipid Res.* **32**:1265-1273, 1991).

In *Euglena* the products of Δ⁸-desaturation, 20:3Δ^{8,11,14} and 20:4Δ^{8,11,14,17}, may be incorporated directly into membranes, or subjected to desaturation at the Δ⁵-position to produce arachidonic and eicosapentaenoic acids (Hulanicka et al., *J*. *Biol. Chem.* **239:***2778-*2787, 1964). Further elongation and desaturation leads to several polyunsaturated 22-carbon fatty acids (FIG. 2). In mammals, whether the products are derived from Δ⁸ or Δ⁶ activity, similar processes produce mostly arachidonic, eicosapentaenoic, and docosahexaenoic acid (22:6 (Hwang, *Fatty Acids in Foods and Their Health Implications,* pp. 545-557, 1992; Bernert and Sprecher, *Biochim. Biophys. Acta* **398**:354-363, 1975; Lees and Korn, *Biochemistry* **5**:1475-1481, 1966; Albert and Coniglio, *Biochim. Biophys. Acta* **489**:390-396, 1977; Bardon et al., *Cancer Lett.* **99**:51-58, 1996; and Sprecher and Lee, *Biochim.Biophys.Acta.* **388**:113-125, 1975), although some 20:3 is metabolized directly to series 1 eicosanoid metabolic regulators (Hwang, *Fatty Acids in Foods and Their Health Implications,* pp. 545-557, 1992).

It is interesting to note that the alternate pathway of Δ⁸-desaturation begins with an elongation step. This elongation is the standard pathway in *Euglena,* which produces substantial amounts of 20:2 (7.4%) and 20:3 (1.4%) (FIG. 2). In mammalian tissue with little or no Δ⁶ activity (Grammatikos et al., *Br. J. Cancer* **70:**219-227, 1994), this would be the first step by which the essential fatty acids 18:2 and 18:3 are metabolized to their 20-carbon derivatives. Recently there has been a new emphasis on fatty acid chain elongation acting as a regulatory step in fatty acid biosynthesis (Garcia et al., *Lipids* **25:**211-215, 1990; Sprecher et al., *Prostag. Leukot Essent. Fatty* Acids **52:**99-101, 1995). Evidence that breast cancer cells may selectively elongate 18:3 in preference to 18:2, and that Δ⁸-desaturation follows this elongation (Bardon et al., *Cancer Lett.* **99:**51-58, 1996) implies that Δ⁸-desaturation may play an important role in some cancer cells.

The identification and cloning of a Δ⁸-desaturase gene permit examinations of the alternate pathway for biosynthesis of 20-carbon polyunsaturated fatty acids and will give insight into the possible mechanisms of Δ⁸ -desaturation. In mammals, operation of this alternative pathway may be confined to specialized tissues, where the demand for polyunsaturated fatty exceeds the supply provided through rate-limiting Δ⁶ desaturation. The pathway may be of greater significance where Δ⁶ desaturation is reduced or absent Since fatty acid desaturase metabolism is perturbed in many cell lines, both transformed (Grammatikos et al., *Ann. N. Y. Acad. Sci.* **745**:92-105,1994) and untransformed (Rosenthal, *Prog. Lipid Res.* **26**:87-124, 1987), it may be that Δ⁸ activity is only revealed in the absence of Δ⁶ activity. Alternatively, Δ⁸ activity may arise or increase with cell neoplasia. The isolation and examination of this Δ⁸ gene, and analysis of its substrate specificity, should facilitate the determination of the role of Δ⁸ activity in both normal and cancerous mammalian tissue.

### Comparative Example 14: Discussion of the C. elegans Δ⁵-desaturase.

In this example, we describe a region of the *C. elegans* genome located at position 4.88 of chromosome IV is described that contains the Δ⁵ - and Δ⁶ - desaturase genes. The amino acid sequences encoded by the two genes are 46% identical to each other, and each contains an N-terminal heme binding domain typical of the electron carrier cytochrome *b*₅ and three histidine boxes. Both genes contain the consensus sequence of the third His box (QXXHH; SEQ. ID NO. 11) that has so far been shown to be unique to the microsomal desaturases involved in double-bond insertion at carbons below position 9.

Despite these similarities these two microsomal desaturases show absolutely non-overlapping substrate specificities. When overexpressed in the yeast *Saccharomyces* cerevisiae, the *C*. *elegans* Δ⁶-desaturase (FAT-3) specifically acts on two 18-carbon substrates, linoleic and γ-linolenic acid, and always desaturates in a methylene-interrupted pattern (one double bond every three carbons). The mammalian Δ⁶-desaturase system has likewise been demonstrated to insert double bonds strictly in a methylene-interrupted pattern and to have no activity on 20-carbon substrates (Schmitz et al., *Lipids* **12**:307-313, 1997). The *C*. *elegans* Δ⁵ -desaturase (FAT-4), in contrast, acts on a number of 20-carbon substrates, as well as on an endogenous 18:1 fatty acid of yeast, and is capable of inserting double bonds in a non-methylene interrupted pattern.

Non-methylene-interrupted fatty acids such as 20:2 Δ^{5,11}, 20:3 Δ^{5,11,14}, 18:2 Δ^{5,11} have been detected in mammalian cells by feeding ¹⁴C-labeled substrates to rats raised on a fat-deficient diet (Ulman et al., *Biochem. Biophys. Acta* **248**:186-197,1971). However, these fatty acids are considered to be "dead end" metabolites, as they have not been demonstrated to serve as precursors to signaling molecules such as prostaglandins, nor are they detectable in tissue lipids of rats who are not preconditioned on a fat-deficient diet. (We also did not detect these fatty acids in *C. elegans* lipid extracts.)

In yeast expressing the *C. elegans* Δ*⁵*-desaturase gene, the amount of substrate converted was greatest for the metabolically significant substrate 20:3 Δ^{8,11,14} (FIG. 13). The amount of 20:2Δ^{11,14} and 20:3Δ^{11,14,17} that was desaturated less than half the amount of conventional substrate that was desaturated. This was consistent with the rates of desaturation in microsomal extracts of mammalian liver, where the rate of conversion of labeled 20:2Δ^{11,14} to 20:3Δ^{5,11,14} is 41 % of the rate of conversion of labeled 20:3Δ^{8,11,14} to 20:4Δ^{5,8,11,14} (Bernet et al., *Biochem. Biophys. Acta* **398**:354-313, 1975).

The *C. elegans fat-3* and *fat-4* genes are present in a gene cluster in the same 5' to 3' orientation. Yet, unlike other gene clusters of this sort in *C. elegans,* the downstream *fat-3* gene is not transpliced to SL2, and therefore is unlikely to be co-transcribed with the upstream *fat-4* gene. The two genes could be located next to each other as a result of an ancient gene-duplication event. The DNA sequences share 54% identity over the entire cDNA coding sequence; however the genes do not share any common intron/exon boundaries (FIG. 8).

This is the first disclosed sequence of a Δ⁵ -desaturase gene from an animal. The sequence of the *C. elegans* Δ⁵ -desaturase is quite distant from the bacterial and fungal Δ⁵-desaturases that have been reported, and this animal sequence should facilitate the search for desaturase-encoding sequences from humans and other mammals. Both the Δ⁵ - and Δ⁶-desaturases are important regulatory enzymes in humans. They participate in critical steps in the pathway to produce precursors for synthesis of hormone-like eicosanoid molecules from the essential dietary fatty acids, linoleic acid and α-linolenic acid. The activities of these desaturases have been shown to be under hormonal and nutritional control, but the mechanism of this control is still unknown.

Certain diseases, such as diabetes, result in low Δ⁵-desaturase activity, while HTC cells, isolated from an ascites tumor derived from a solid hepatoma, show increased Δ⁵-desaturase activity. The availability of mutational and reverse genetic tools and the expanding knowledge of cellular and developmental biology in *C. elegans* make this an attractive system to study the roles of polyunsaturated fatty acids and their metabolic products in development, reproduction, and other cellular processes of animals.

### Example 15: A Plant Cell Transformed with the Δ⁵ and Δ⁸ -desaturase Genes of the Invention

Using the methods described herein, Δ⁵ - and Δ⁸ -desaturases of the invention may be cloned and expressed in plants to produce plants with enhanced amounts of 20-carbon polyunsaturated fatty acids. Such plants provide an inexpensive and convenient source of these important fatty acids in a readily harvestable and edible form.

For instance, the Δ⁵ - and Δ⁸ -desaturases of the invention can be cloned into a common food crop, such as corn, wheat, potato, tomato, yarns, apples, pears, or into oil-seed plants such as sunflower, rapeseed, soy, or peanut plants. The resulting plant would express the appropriate enzyme that would catalyze the formation of 20-carbon polyunsaturated fatty acids. In the case of an oil-seed plant, the seed oil would be a rich source of 20-carbon polyunsaturated fatty acids.

The Δ⁵ - and Δ⁸ -desaturase genes may be cloned and expressed either individually, or together in a host plant cell. The corresponding desaturases can be expressed using a variety of different control sequences, such as promoters, enhancers, and 3'-termination sequences. These control sequences can be used to control the expression of each desaturase individually. For example, the Δ⁵ -desaturase can be cloned such that it is under the control of a strong promoter, and the Δ⁸ -desaturase can be cloned such that it is under the control of a weak promoter, thus yielding a transgenic plant that expresses more Δ⁵ -desaturase than Δ⁸ -desaturase. Furthermore, expression can be controlled by operably linking one or more of the desaturase genes of interest to a promoter that is activated by exposure of the plant cell to an appropriate regulatory agent such as an inducer, repressor, de-repressor or inhibitor agent. Such regulation is discussed above. Alternatively, expression of non-contiguous genes may be coordinated by linking the expression of a first gene with the expression of an inducer or de-repressor molecule that induces or de-represses the expression of a second gene.

The genes of the invention can be integrated into the genome of a plant (for example, by *Agrobacterium-mediated* T-DNA transfer) or animal (for example, by use of broad host-range retroviruses, e.g., an adenovirus vector) so that the Δ⁵ and Δ⁸ -desaturases of the invention are expressed as part of the genome. For transgenic plants, the T-DNA vector may be used that would result in integration of transgenes (Δ⁵ and/or Δ⁸) into the host cell genome.

For example, the expression of the Δ⁵ -and Δ⁸ - desaturases in a plant, such as *Arabidopsis,* can be achieved by constructing a plant transformation vector to introduce the cDNA of each desaturase into the plant. The vector can contain a tissue specific promoter so that the desaturase protein will be expressed during seed development. Examples of seed specific promoters include that for phaseolin (van der Geest and Hall, *Plant Mol. Biol.* **32:**579-88, 1996) or the promoter for napin (Stalberg et al., *Plant Mol. Biol*. 23: 671-83, 1993). Other seed-specific promoters that can be used are those located on the genomic BAC clone T24A18 (LOCUS ATT24A18. (1999) 45980 bp *Arabidopsis thaliana* DNA chromosome 4,ACCESSION # AL035680, NID g4490701) of the *Arabidopsis* genome. These promoters regulate seed storage protein expression in *Arabidopsis.* Other promoters which express genes specifically in seeds like those described in (Parcy et al., *Plant Cell* 6:1567-1582, 1994) can also be used. The constructs containing the desaturase coding sequence and promoter sequence can then transferred to standard plant transformation T-DNA vectors similar to pART27 (Gleave, *Plant Mol. Biol.* 20:1203-1207, 1992), pGPTV (Becker et al., *Plant Mol. Biol.* **20**:1195-7, 1992), or pJIT119 (Guerineau et al., *Plant Mol. Biol.* **15**:127-136, 1992). If the plant is to be transformed with two constructs, i.e. one encoding the Δ⁸-desaturase and the other encoding the Δ⁵-desaturase, then it is preferable to choose two different selectable markers so that only double transformants will regenerate. For example, the vector carrying the Δ⁵-desaturase can be constructed such that it contains the kanmycin *(npt*II) gene, and the vector carrying the Δ⁸-desaturase can be constructed such that it contains the phosphinothricin (bar) gene. Transformants are then selected on media containing kanmycin and phosphinothricin. Transformation of Arabidopsis is readily achieved using the Agrobacterium-mediated vacuum infiltration process (Katavic et al., *Mol.* Gen. *Genet.* **245**:363-70, 1994) or the floral dip modification of it (Clough and Bent, *Plant J.* 16:735-43, 1998), although several other methods are also commonly used. Transgenic progeny will be identified by selection using the appropriate antibiotic or herbicide, either kanmycin or phosphinothricin, or both. Since the Δ⁸ and Δ⁵ constructs use different selectable markers the double transformants are readily isolated. Plants which survive the transgenic selection are grown to maturity and their seed harvested. The seeds of transformed plants are analyzed by isolation of fatty acid methyl esters followed by gas chromatography to determine the fatty acid composition.

Plants expressing only the Δ⁸-desaturase will desaturate the 20:1Δ¹¹ fatty acid that occurs naturally in the *Arabidopsis* seed to 20:2Δ^{8,11}. Seed harvested from plants doubly transformed with both desaturases will, in addition, convert the 20:2Δ^{8,11} product of the Δ⁸-desaturase plants to 20:3Δ^{5,8,11} as a result of the expression of the Δ⁵-desaturase. These changes will be easily detected by the fatty acid methyl ester analysis.

### Example 16: A Yeast Cell Transformed with the Δ⁵ - and Δ⁸ -desaturase Genes of the Invention

The cDNA portion of pJW541 (Wallis and Browse, *Arch. Biochem. Biophys.* **365:**307-316, 1999) containing the *Euglena* Δ⁸ -desaturase was excised from that plasmid with the restriction enzymes EcoRI and Spel. The purified DNA fragment representing the insert was ligated into the yeast expression vector pYX232 (R&D Systems, Inc.) that had been prepared by digestion with EcoRI and Nhel, to give compatible sticky ends. (Plasmid pYX232 carries the marker conferring yeast prototrophy for tryptophan (TRP1 mutation), and uses the triose phosphate isomerase (TPI) promoter for constitutive expression of the inserted DNA.) The resulting plasmid, pYX232-541, was introduced into the *Saccharomyces cerevisiae* strain already harboring the Δ⁵ -desaturase (pYFAT4; Watts and Browse, *Arch. Biochem. Biophys.* **362**:175-182, 1999) plasmid that confers yeast prototrophy for uracil using a lithium acetate transformation procedure (Invitrogen). Transformants were selected simultaneously for uracil and tryptophan prototrophy. Selected colonies arising after the transformation were inoculated into yeast minimal medium that also lacked both uracil and tryptophan.

For analysis of activity, separate cultures were supplemented with one of three fatty acid substrates provided as sodium salts as described (Wallis and Browse, *Arch. Biochem. Biophys.* **365:**307-316, 1999). After overnight culture at 28°C, the cultures were harvested by centrifugation and washed. Fatty acid methyl esters were prepared using the standard methods described in Miquel and Browse *J. Biol. Chem.* **267**:1502-1509, 1992.

Analysis by gas chromatography indicated that each substrate had been desaturated twice. The incorporation of the three substrates varied, with more unsaturated substrates becoming a greater part of the fatty acid composition of the cells, as seen in other experiments (Wallis and Browse, *Arch. Biochem. Biophys.* **365**:307-316, 1999, and Watts and Browse, *Arch. Biochem. Biophys.* **362:**175-182, 1999. For the tri-unsaturated substrate 20:3Δ^{11,14,17}, the 20-carbon fatty acid represented 37% of the total cellular fatty acid, for 20:2Δ^{11,14} the 20-carbon fatty acid level 21%, and for 20:1Δ¹¹ the 20-carbon fatty acid level reached only 13%. However, the activities of the desaturases were substantially identical against all three substrates. Between 70 and 72% of the substrate was not converted, and 17 or 18% underwent a single desaturation by only one of the enzymes. However, for each substrate, between 11 and 13% of the substrate was desaturated by both enzymes acting in concert to produce a fatty acid with two double bonds more than in molecules of the supplied substrate.

**TABLE 2**

| | | | | | |
|---|---|---|---|---|---|
| **Fatty acid supplement** | **Fatty acid uptake*** | **Unconverted substrate #** | **One added desaturation#** | **Doubly desaturated product #** | |
| 20:3 (11.14.17) | 37 | 71 | 17 | 11 | 20:5(5,8,11,14,17) |
| 20:2 (11,14) | 21 | 70 | 18 | 13 | 20:4 (5,8,11,14) |
| 20:1 (11) | 13 | 72 | 18 | 11 | 20:3 (5,8,11) |

| | | | | | |
|---|---|---|---|---|---|
| *as mass percent of whole cell fatty acids #as mass percent of incorporated 20-carbon fatty acids | | | | | |

The foregoing embodiments and examples are provided only as examples and are in no way meant to limit the scope of the claimed invention.

## Claims

1. A purified protein having desaturase activity, and comprising an amino acid sequence selected from the group consisting of:
(a) an amino acid sequence as shown in SEQ DD NO: 4; and
(b) an amino acid sequence having at least 60% sequence identity to the sequence specified in (a).

2. A desaturase protein as claimed in claim 1, having at least 70% sequence identity with SEQ ID NO: 4.

3. A desaturase protein as claimed in claim 1, having at least 80% sequence identity with SEQ ID NO:4.

4. An isolated nucleic acid molecule encoding the protein according to any one of claims 1 to 3.

5. The isolated nucleic acid molecule of claim 4, comprising a sequence as shown in SEQ ID NO: 3.

6. An isolated nucleic acid molecule according to claim 4, that hybridizes under low-stringency conditions with a nucleic acid probe, the probe comprising a sequence selected from the sequence as shown in SEQ ID NO: 3, and fragments thereof of at least 600 base pairs in length.

7. An isolated nucleic acid molecule according to claim 6, that has at least 60% sequence identity with a nucleic acid sequence as shown in SEQ ID NO: 3.

8. A nucleic acid molecule according to claim 6, having at least 70% sequence identity with SEQ ID NO: 3.

9. A recombinant nucleic acid molecule, comprising a control sequence operably linked to the nucleic acid molecule of any one of claims 4 to 8.

10. A cell transformed with the recombinant nucleic acid molecule of claim 9.

11. A cell according to claim 10 further transformed with a nucleic acid molecule selected from the group consisting of:
(a) a nucleic acid molecule as shown in SEQ ID NO: 1; and
(b) a nucleic acid molecule that has 60% sequence identity to the nucleic acid molecule shown in (a).

12. The cell of claim 10 or 11, wherein the cell is a plant cell.

13. A non-human transgenic organism, comprising the transformed cell of claim 10 or 11, wherein the transgenic organism is selected from the group consisting of plants, bacteria, insects, fungi, and mammals.

14. A method of identifying a nucleic acid sequence, comprising:
(a) hybridizing the nucleic acid sequence to at least 15 contiguous nucleotides of a sequence as shown in SEQ ID NO: 3; and
(b) identifying the nucleic acid sequence as one that encodes a desaturase.

15. The method of claim 14, comprising hybridizing the nucleic acid sequence to at least 20 contiguous nucleotides of a sequence as shown in SEQ ID NO: 3.

16. The method of claim 14 or 15, wherein hybridizing the nucleic acid sequence is performed under low-stringency conditions.

17. The method of claim 14, wherein step (a) occurs in a PCR reaction.

18. The method of claim 14, wherein step (a) occurs during a library screening.

19. A method for creating a double bond between two carbons in a fatty acid, comprising:
contacting a fatty acid with at least one purified desaturase of claim 1; and
allowing the desaturase to create a double-bond between two carbons.

20. The method of claim 19, wherein the desaturase is expressed in a non-human transgenic organism and the double-bond formation occurs *in vivo.*

21. The method of claim 20, wherein the desaturase is expressed in a non-human organism selected from the group consisting of eukaryotes and prokaryotes.

22. The method of claim 19, wherein the desaturase is expressed *in vitro* and the double-bond formation occurs *in vitro.*

23. The method of claim 19, further comprising expressing a second desaturase.

24. The method of claim 23, wherein the second desaturase is selected from the group consisting of:
(a) an amino acid sequence as shown in SEQ ID NO: 2;
(b) an amino acid sequence that differs from those specified in (a) by one or more conservative amino acid substitutions; and
(c) an amino acid sequence having at least 60% sequence identity to a sequence specified in (a) or (b).

## Patentansprüche

1. Gereinigtes Protein, das eine Desaturase-Aktivität aufweist und eine Aminosäuresequenz, ausgewählt aus der Gruppe bestehend aus:
a) einer Aminosäuresequenz wie in SEQ ID NO: 4 gezeigt; und
b) einer Aminosäuresequenz, die mindestens 60 % Sequenzidentität zu der in a) angegebenen Sequenz aufweist
umfaßt.

2. Desaturase-Protein wie beansprucht in Anspruch 1, das mindestens 70 % Sequenzidentität zu SEQ ID NO: 4 aufweist.

3. Desaturase-Protein wie beansprucht in Anspruch 1, das mindestens 80 % Sequenzidentität zu SEQ ID NO: 4 aufweist.

4. Isoliertes Nukleinsäuremolekül, das für ein Protein nach einem der Ansprüche 1-3 kodiert.

5. Isoliertes Nukleinsäuremolekül nach Anspruch 4, umfassend eine Sequenz wie in SEQ ID NO: 3 gezeigt.

6. Isoliertes Nukleinsäuremolekül nach Anspruch 4, das unter Bedingungen niedriger Stringenz mit einer Nukleinsäure-Sonde hybridisiert, wobei die Sonde eine Sequenz, ausgewählt aus der Sequenz wie in SEQ ID NO: 3 gezeigt, umfaßt, und Fragmente davon von mindestens 600 Basenpaaren Länge.

7. Isoliertes Nukleinsäuremolekül nach Anspruch 6, das mindestens 60 % Sequenzidentität zu einer Nukleinsäuresequenz wie in SEQ ID NO: 3 gezeigt aufweist.

8. Nukleinsäuremolekül nach Anspruch 6, das mindestens 70 % Sequenzidentität mit SEQ ID NO: 3 aufweist.

9. Rekombinantes Nukleinsäuremolekül, umfassend eine Kontrollsequenz, die operativ mit dem Nukleinsäuremolekül nach einem der Ansprüche 4-8 verbunden ist.

10. Zelle, transformiert mit dem rekombinanten Nukleinsäuremolekül nach Anspruch 9.

11. Zelle nach Anspruch 10, weiterhin transformiert mit einem Nukleinsäuremolekül, ausgewählt aus der Gruppe bestehend aus:
a) einem Nukleinsäuremolekül wie in SEQ ID NO: 1 gezeigt; und
b) einem Nukleinsäuremolekül, das mindestens 60 % Sequenzidentität zu dem Nukleinsäuremolekül wie in a) gezeigt aufweist.

12. Zelle nach Anspruch 10 oder 11, wobei die Zelle eine Pflanzenzelle ist.

13. Nicht-menschlicher transgener Organismus, umfassend die transformierte Zelle nach Anspruch 10 oder 11, wobei der transgene Organismus ausgewählt ist aus der Gruppe bestehend aus Pflanzen, Bakterien, Insekten, Pilzen und Säugern.

14. Verfahren zur Identifizierung einer Nukleinsäuresequenz, umfassend:
a) Hybridisieren der Nukleinsäuresequenz an mindestens 15 kontinuierliche Nukleotide einer Sequenz wie in SEQ ID NO: 3 gezeigt; und
b) Identifizieren der Nukleinsäuresequenz als eine, die für eine Desaturase kodiert.

15. Verfahren nach Anspruch 14, umfassend ein Hybridisieren der Nukleinsäuresequenz an mindestens 20 kontinuierliche Nukleotide einer Sequenz wie in SEQ ID NO: 3 gezeigt.

16. Verfahren nach Anspruch 14 oder 15, wobei das Hybridisieren der Nukleinsäuresequenz unter Bedingungen niedriger Stringenz durchgeführt wird.

17. Verfahren nach Anspruch 14, wobei Schritt a) während einer PCR-Reaktion stattfindet.

18. Verfahren nach Anspruch 14, wobei Schritt a) während eines Bibliotheks-Screenings auftritt.

19. Verfahren zur Erzeugung einer Doppelbindung zwischen zwei Kohlenstoffen in einer Fettsäure, umfassend:
In-Kontakt-Bringen einer Fettsäure mit mindestens einer gereinigten Desaturase nach Anspruch 1; und
Ermöglichen der Desaturase, eine Doppelbindung zwischen zwei Kohlenstoffen zu erzeugen.

20. Verfahren nach Anspruch 19, wobei die Desaturase in einem nicht-menschlichen transgenen Organismus exprimiert wird und die Doppelbindungs-Bildung *in vivo* auftritt.

21. Verfahren nach Anspruch 20, wobei die Desaturase in einem nicht-menschlichen Organismus exprimiert wird, ausgewählt aus der Gruppe bestehend aus Eukaryonten und Prokaryonten.

22. Verfahren nach Anspruch 19, wobei die Desaturase *in vitro* exprimiert wird und die Doppelbindungs-Bildung *in vitro* auftritt.

23. Verfahren nach Anspruch 19, weiterhin umfassend Exprimieren einer zweiten Desaturase.

24. Verfahren nach Anspruch 23, wobei die zweite Desaturase ausgewählt ist aus der Gruppe bestehend aus:
a) einer Aminosäuresequenz wie in SEQ ID NO: 2 gezeigt;
b) einer Aminosäuresequenz, die sich von der in a) angegebenen durch eine oder mehrere konservative Aminosäuresubstitutionen unterscheidet; und
c) einer Aminosäuresequenz, die mindestens 60 % Sequenzidentität zu einer in a) oder b) angegebenen Sequenz aufweist.

## Revendications

1. Protéine purifiée possédant une activité de désaturase et comprenant une séquence d'acides aminés choisie dans l'ensemble constitué par les suivantes :
a) la séquence d'acides aminés présentée en tant que Séquence n° 4.
b) et une séquence d'acides aminés identique, pour au moins 60 %,
à la séquence indiquée en (a).

2. Protéine désaturase conforme à la revendication 1, dont la séquence est identique, pour au moins 70 %, à la Séquence n° 4.

3. Protéine désaturase conforme à la revendication 1, dont la séquence est identique, pour au moins 80 %, à la Séquence n° 4.

4. Molécule d'acide nucléique isolée, codant une protéine conforme à l'une des revendications 1 à 3.

5. Molécule d'acide nucléique isolée, conforme à la revendication 4, comprenant la séquence présentée en tant que Séquence n° 3.

6. Molécule d'acide nucléique isolée, conforme à la revendication 4, qui s'hybride dans des conditions de faible stringence avec une sonde d'acide nucléique, laquelle sonde comprend une séquence choisie parmi la séquence présentée en tant que Séquence n° 3 et ses fragments longs d'au moins 600 paires de bases.

7. Molécule d'acide nucléique isolée, conforme à la revendication 6, dont la séquence est identique, pour au moins 60 %, à la séquence présentée en tant que Séquence n° 3.

8. Molécule d'acide nucléique isolée, conforme à la revendication 6. dont la séquence est identique, pour au moins 70 %, à la Séquence n° 3.

9. Molécule d'acide nucléique recombinée, comprenant une séquence régulatrice opérationnellement liée à une molécule d'acide nucléique conforme à l'une des revendications 4 à 8.

10. Cellule transformée avec une molécule d'acide nucléique recombinée conforme à la revendication 9.

11. Cellule conforme à la revendication 10, transformée en outre avec une molécule d'acide nucléique choisie parmi les suivantes :
a) la Molécule d'acide nucléique dont la séquence est présentée en tant que Séquence n° 1.
b) et une molécule d'acide nucléique dont la séquence est identique, pour au moins 60 %, à celle de la molécule d'acide nucléique indiquée en (a).

12. Cellule conforme à la revendication 10 ou 11, laquelle cellule est une cellule végétale.

13. Organisme transgénique non-humain, comprenant une cellule transformée conforme à la revendication 10 ou 11, lequel organisme transgénique est choisi dans l'ensemble constitué par les plantes, bactéries, insectes, champignons et mammifères.

14. Procédé d'identification d'une séquence d'acide nucléique, qui comprend :
a) le fait d'hybrider cette séquence d'acide nucléique avec au moins 15 nucléotides contigus de la séquence présentée en tant que Séquence n° 3,
b) et le fait d'identifier cette séquence d'acide nucléique comme étant une séquence codant une desaturase.

15. Procédé conforme à la revendication 14, qui comprend le fait d'hybrider ladite séquence d'acide nucléique avec au moins 20 nucléotides contigus de la séquence présentée en tant que Séquence n° 3.

16. Procédé conforme à la revendication 14 ou 15, dans lequel on effectue l'hybridation de ladite séquence d'acide nucléique dans des conditions de faible stringence.

17. Procédé conforme à la revendication 14, dans lequel l'étape (a) se déroule au cours d'une réaction d'amplification en chaîne par polymérase (PCR).

18. Procédé conforme à la revendication 14, dans lequel l'étape (a) se déroule au cours d'un criblage de banque.

19. Procédé de formation d'une double liaison entre deux atomes de carbone chez un acide gras, qui comprend :
- le fait de mettre un acide gras en contact avec au moins une désaturase purifiée conforme à la revendication 1,
- et le fait de laisser la désaturase former une double liaison entre deux atomes de carbone.

20. Procédé conforme à la revendication 19, dans lequel la désaturase est exprimée chez un organisme transgénique non-humain et la formation d'une double liaison a lieu *in vivo.*

21. Procédé conforme à la revendication 20, dans lequel la désaturase est exprimée chez un organisme non-humain choisi parmi les eucaryotes et les procaryotes.

22. Procédé conforme a la revendication 19, dans lequel la désaturase est exprimée *in vitro* et la formation d'une double liaison a lieu *in vitro.*

23. Procédé conforme à la revendication 19, qui comporte en outre l'expression d'une deuxième désaturase.

24. Procédé conforme à la revendication 23, dans lequel la deuxième désaturase est choisie parmi les protéines présentant :
a) la séquence d'acides aminés présentée en tant que Séquence n° 2,
b) une séquence d'acides aminés qui diffère de celle indiquée en (a) par une ou plusieurs substitutions conservatives d'acides aminés,
c) ou une séquence d'acides aminés identique, pour au moins 60 %, à la séquence indiquée en (a) ou (b).
